# FASCICULE DE BREVET EUROPEEN

(11) **EP 3 416 730 B1**
(45) Date de publication et mention de la délivrance du brevet: **07.07.2021**
(21) Numéro de dépôt: 17710598.8
(22) Date de dépôt: 20.02.2017
(51) Int. Cl.: A61Q 19/08, A61Q 19/00, C07K 5/10, A61K 8/64, A61K 8/06, A61K 47/54

(54) **COMPOSITION COSMÉTIQUE CUTANÉE COMPRENANT UN PEPTIDE SDKP OU UN ANALOGUE DE CELUI-CI**
DERMATOLOGISCHE KOSMETISCHE ZUSAMMENSETZUNG MIT EINEM SDKP-PEPTID ODER EINEM ANALOGON DAVON
DERMATOLOGICAL COSMETIC COMPOSITION COMPRISING AN SDKP PEPTIDE OR AN ANALOG THEREOF

(30) Priorité: 18.02.2016 FR 1651328
(43) Date de publication de la demande: 26.12.2018
(73) Titulaire: RPM Dermatologie, 30400 Villeneuve Les Avignon (FR)
(72) Inventeur: RIVIERE, Nicolas, 30400 Villeneuve Les Avignon (FR)
(74) Mandataire: Plasseraud IP
(86) Numéro de dépôt international: PCT/FR2017/050373
(87) Numéro de publication internationale: WO 2017/140997

(56) Documents cités:
- US-A- 4 171 299
- US-A1- 2010 098 752
- US-A1- 2015 202 139
- JOSIANE THIERRY ET AL: "Synthesis and biological evaluation of analogues of the tetrapeptideN-acetyl-Ser-Asp-Lys-Pro (AcSDKP), an inhibitor of primitive haematopoietic cell proliferation", JOURNAL OF PEPTIDE SCIENCE., vol. 7, no. 5, 1 mai 2001 (2001-05-01), pages 284-293, XP055292398, GB ISSN: 1075-2617, DOI: 10.1002/psc.322

## Description

### INTRODUCTION

L'objet de la présente invention concerne une néogoutte (c'est-à-dire une nanoparticule lipidique) comprenant un conjugué peptidique SDKP, un procédé d'obtention de cette néogoutte, ainsi que son utilisation cosmétique, de préférence topique, en tant qu'agent antirides ou restructurant de la peau. Plus particulièrement, l'objet de la présente invention concerne un conjugué peptidique SDKP pour une telle utilisation.

Le vieillissement de la peau est un phénomène complexe ayant des origines diverses, qui peuvent être extérieures (exposition au soleil, au froid, agents oxydants, etc.) ou intrinsèques (génétique, protéiques, etc.).

La peau est un tissu, constitué de couches (épiderme, derme, et hypoderme, derme et épiderme étant liés par la jonction dermo-épidermique « JDE »), ayant le rôle de barrière de protection entre le milieu intérieur et extérieur.

Le vieillissement de la peau se traduit en surface par l'apparition de rides et ridules. Ceci est dû en partie par un relâchement des tissus cutanés et sous cutanés et à une perte d'élasticité de ces derniers. Ainsi, le derme assure la fonction fondamentale de cohésion de la peau et constitue une cible privilégiée du traitement antirides.

La perte d'élasticité du derme/épiderme s'explique par la conjoncture de plusieurs phénomènes. Elle est due en partie par la diminution de la quantité de collagène et la réduction du taux de fibronectine dans le derme. De plus, les fibroblastes se transforment en fibrocytes ou disparaissent. A ceci, s'ajoute un aplatissement de la JDE qui perd ainsi progressivement ses ondulations caractéristiques diminuant en conséquence l'interface épiderme-derme. Les réseaux moléculaires de la peau se déstructurent : le squelette protéique se fragilise, les kératinocytes basaux montrent une adhérence moindre, ce qui a pour conséquence une altération des fonctions biologiques et de soutien de la JDE.

Des moyens variés ont été proposés contre le vieillissement cutané, allant de la protection solaire au renforcement de la JDE en passant par l'activation de la régénération cellulaire.

La molécule Ac-SDKP-OH est un tétrapeptide naturel (Acétyl- Sérine-Acide aspartique-Lysine-Proline-OH) identifiée pour ses propriétés cosmétiques au niveau de la peau (EP 1 786 386, Bakala et al.).

Toutefois, cette molécule s'hydrolyse très facilement, ce qui rend son exploitation commerciale délicate en particulier pour des compositions cosmétiques d'usage commun (i.e. stockées à température ambiante, ouvertes et refermées à la convenance du patient). La molécule est en outre très sensible à l'hydrolyse enzymatique. Il en est de même pour ses analogues couverts par EP 1786 386. Ainsi, de par sa fragilité, l'accès aux couches les plus profondes de la peau par cette molécule est limité, restreignant ainsi l'effet du traitement. En effet, comme expliqué ci-dessus, le vieillissement de la peau est dû en partie à des altérations de ses couches plus profondes (dermique en particulier). Une solution envisagée à ceci a été de chercher à limiter l'hydrolyse enzymatique en ajoutant des groupements sur la molécule. Effectivement, d'une manière générale plus on « encombre » autour du peptide, moins on facilite l'hydrolyse par l'enzyme. Néanmoins, dans ce cas, lorsque AcSDKP a été substitué, en laboratoire un kit de dosage ELISA ne fonctionnait plus impliquant que la molécule avait perdu son activité au moins partiellement. Ainsi, la substitution non-contrôlée du peptide AcSDKP ne permet pas de surmonter le problème technique lié à sa fragilité, même si le produit nu est actif en surface de la peau.

Ainsi, il a été envisagé d'utiliser une formulation de type émulsion/nanoémulsion pour surmonter ces difficultés. En effet, il est connu que la phase lipidique d'une émulsion permet une pénétration améliorée dans la peau de principes actifs.

Le document brevet FR 2 934 955 divulgue l'encapsulation d'agents thérapeutiques lipophiles ou amphiphiles dans des nanoémulsions. Il est divulgué dans FR 2 934 955 des nanocapsules fonctionnalisées par un cyclopeptide (cRGD) couplé à un co-tensioactif de greffage, le distéaroylophosphatidyléthalomaine poly(éthylène glycol) 5000 maléimide, ce qui montre que ce type de nanoémulsion est tout à fait compatible avec des peptides. Toutefois, le fragment peptidique étant hydrophile, il se positionne dans la phase aqueuse de l'émulsion, là où il est susceptible de subir une hydrolyse, laquelle hydrolyse n'est absolument pas désirée.

Le document brevet FR 2 991 196 divulgue des nanoparticules ciblantes comprenant un cœur constitué d'une phase lipidique (L1) ou d'une phase aqueuse (A1) ; au moins un agent tensioactif comprenant une partie hydrophile et une partie lipophile ; une membrane interne entourant ledit cœur; une membrane externe entourant ladite membrane interne ; et au moins un ligand de ciblage comprenant une partie lipophile et une partie hydrophile.

Le document Josiane Thierry et al. « Synthesis and biological evaluation of analogues of the tetrapeptideN-acetyl-Ser-Asp-Lys-Pro (AcSDKP), an inhibitor of primitive haematopoietic cell proliferation » Journal of peptide science, vol.7, no.5, 1 mai 2001, pages 284-293 se rapporte à à l'impact des modifications en C terminal ou N terminal ou sein du peptide SDKP sur l'activité antiproliférative des cellules souches hématopoïétiques.

Le document US4171299A se rapporte à de nouveaux polypeptides de faible poids moléculaire, contenant de 3 à 10 acides aminés, utiles comme agents thérapeutiques dans le traitement des maladies allergiques ou du syndrome allergique.

Le document US2010/098752A1 se rapporte à vésicules lipidiques incorporant au moins un agent choisi parmi les antioxydants, les agents anti-inflammatoires, les peptides, les humectants, les agents de protection solaire et les émollients.

Le document US2015202139A1) se rapporte à des formulations cosmétiques antivieillissement applicables localement et pouvant être appliquées sur différentes zones de la peau.

De manière surprenante, la Demanderesse s'est aperçue que la stabilité et le profil d'action de la molécule « AcSDKP » pouvait être améliorés si le peptide était modifié pour être incorporé dans des systèmes tels que décrits dans FR 2 934 955 ou encore FR 2 991 196 en remplaçant l'acétyle en N-terminale par une chaîne grasse, ou en ajoutant une chaîne grasse en C-terminale. Ce qui est particulièrement surprenant dans la présente invention, c'est que la Demanderesse a employé cette technologie, en particulier celle du brevet FR 2 991 196, non pas pour enfouir le peptide dans la phase huileuse, là où la/les enzymes ne peuvent accéder, mais dans la phase aqueuse, accessible. En particulier dans l'utilisation des nanoparticules selon FR 2 991 196, au lieu d'encapsuler le peptide SDKP dans le cœur de la nanoparticule (pouvant être une phase aqueuse), la Demanderesse a formé une nanoparticule au cœur lipidique, et a greffé une chaîne grasse à SDKP pour l'incorporer dans la nanoparticule de manière à ce que SDKP soit disposé vers l'extérieur de la nanoparticule. La Demanderesse s'est aperçue alors qu'en traitant des échantillons de peau, de manière surprenante, la couche dermique de la peau était atteinte, alors que le peptide aurait dû être dégradé avant d'atteindre cette couche. L'activité de SDKP sur la peau a été augmentée de manière significative lorsque SDKP est placé vers l'extérieur d'une néogoutte au coeur lipidique comprenant au moins un agent tensioactif; une membrane interne entourant ledit cœur; et une membrane externe entourant ladite membrane interne. Ainsi, la Demanderesse ne comprend pas encore très bien à quoi est due cette activité. Vraisemblablement l'activité (et/ou l'absence de fragilité) est au moins partiellement liée à la taille de la chaîne grasse greffée sur le brin peptidique SDKP et/ou la disposition originale du peptide dans la phase aqueuse extérieure de la néogoutte. A cet égard, l'enseignement divulgué dans FR 2934955 serait tout à fait utilisable avec les nouvelles molécules SDKP greffées de la présente invention.

### RESUME DE L'INVENTION

L'objet de la présente invention concerne un conjugué peptidique de formule (I) suivante :

R₁-X₁-X₂-X₃-X₄-A₁-R₂ (I)

dans laquelle :
- R₁, étant du côté N-terminal, est un atome d'hydrogène, ou une chaîne grasse choisie parmi un groupe alkyle substitué ou non substitué, linéaire ou ramifié en C₁₃-C₅₀, un groupe alkyle-aryle substitué ou non substitué, linéaire ou ramifié en C₂₁-C₅₀, un groupe alcènyle substitué ou non substitué, linéaire ou ramifié en C₁₃-C₅₀, un groupe R₃-CO-, R₃-OCO- ou R₃-COO- dans lequel R₃ est un groupe alkyle ou alcènyle substitué ou non substitué, linéaire ou ramifié en C₁₃-C₄₉, les substitutions incluant F, Cl, ou tout hétéroatome ou groupe d'hétéroatomes lipophile tel qu'une guanidine ou un guanidinium,
- X₁ est un condensat de (L)-sérine,
- X₂ est un condensat de (L) et/ou (D) acide aspartique,
- X₃ est un condensat de (L) et/ou (D) lysine,
- X₄ est un condensat de (L) et/ou (D) proline,
- X₄ étant optionnellement absent,
- les liaisons reliant X₁ à X₂, X₂ à X₃ le cas échéant, et X₃ à X₄ le cas échéant, pouvant être des liaisons peptidiques ou pseudopeptidiques,
- A₁ est une liaison covalente, un groupement NH ou un atome d'oxygène,
- R₂ est un atome d'hydrogène, ou une chaîne grasse choisie parmi un groupe alkyle substitué ou non substitué, linéaire ou ramifié en C₁₃-C₅₀, un groupe alkyle-aryle substitué ou non substitué, linéaire ou ramifié en C₂₁-C₅₀, un groupe alcènyle substitué ou non substitué, linéaire ou ramifié en C₁₃-C₅₀, un groupe R₄-CO-, ou R₄-OCO- dans lequel R₄ est un groupe alkyle ou alcènyle substitué ou non substitué, linéaire ou ramifié en C₁₃-C₅₀, les substitutions incluant F, Cl, ou tout hétéroatome ou groupe d'hétéroatomes lipophile tel qu'un groupement guanidine ou guanidinium,
- R₁ et R₂ ne pouvant être conjointement des atomes d'hydrogène,
ou l'un de ses sels pharmaceutiquement acceptables.

L'objet de la présente invention concerne en outre une néogoutte comprenant :
- un cœur constitué d'une phase lipidique (L₁);
- au moins un agent tensioactif comprenant une partie hydrophile et une partie lipophile ;
- une membrane interne entourant ledit cœur constitué d'une phase lipidique (L₂), comprenant la partie lipophile dudit agent tensioactif;
- une membrane externe, entourant ladite membrane interne, constituée d'une phase aqueuse (A₁) comprenant la partie hydrophile dudit agent tensioactif ; et
- au moins un conjugué peptidique de formule (I), caractérisé en ce qu'au moins une chaîne grasse en C₃-C₅₀, préférentiellement en C₄-C₄₀, en C₅-C₃₀, ou encore en C₆-C₂₅, linéaire ou ramifiée, saturée ou insaturée, est greffée à l'extrémité N et/ou C terminale du brin peptidique, par exemple par l'intermédiaire d'un groupement C=O et/ou d'un atome d'oxygène et ledit conjugué peptidique est tel que sa partie lipophile est dans la phase lipidique L₂ et sa partie hydrophile dans la phase A₁.

De plus la présente invention a pour objet un procédé de fabrication d'une néogoutte telle que définie ci-dessus, caractérisé en ce que ledit procédé comprend les étapes suivantes :
a. préparation d'une phase lipidique et d'une phase aqueuse, l'une au moins des deux phases comprenant un agent tensioactif, l'une au moins des deux phases comprenant le brin peptidique de formule SDKP greffé d'une chaîne grasse telle que définie présentement ;
b. émulsification de ladite phase lipidique et de ladite phase aqueuse conduisant à la formation de néogouttes, éventuellement sous pression ;
c. récupération des néogouttes formées.

Ainsi, l'objet de la présente invention concerne une formulation cosmétique comprenant une ou plusieurs néogouttes telles que définies présentement, ladite formulation comprennant préférentiellement entre 1 et 25% en masse de néogouttes selon la présente invention.

En outre, la présente invention concerne une nano-émulsion comprenant :
- au moins une phase lipidique dispersée (L₃), et
- au moins une phase aqueuse continue (A₂) dans laquelle la phase aqueuse comporte au moins un tensioactif,
caractérisée en ce qu'à l'interface entre la phase aqueuse (A₂) et la phase lipidique (L₁) se trouve au moins un conjugué peptidique selon la présente invention et/ou en ce que ladite nano-émulsion comprend au moins une néogoutte selon la présente invention dans laquelle éventuellement les lipides de (L₁) et (L₃), ou (L₂) et (L₃) sont identiques.

L'objet de la présente invention concerne également une utilisation cosmétique de préférence par application topique, d'un conjugué peptidique, d'une néogoutte, d'une nano-émulsion ou d'une formulation cosmétique selon la présente invention pour restructurer ou préserver l'aspect de la peau, en particulier en profondeur au niveau dermique.

De manière préférée, la nano-émulsion ou formulation cosmétique selon la présente invention peut contenir jusqu'à 75% en masse de néogouttes selon la présente invention, préférentiellement entre 1 et 50% en masse de néogouttes selon la présente invention, plus préférentiellement entre 2 et 25% en masse de néogouttes selon la présente invention, encore plus préférentiellement entre 3 et 15% en masse de néogouttes selon la présente invention, , et de manière tout préférée environ 10% (soit entre 9 et 11%) en masse de néogouttes selon la présente invention.

Plus précisément, l'objet de la présente invention concerne ainsi une utilisation cosmétique telle que ci-dessus caractérisée en ce qu'il s'agit d'une utilisation antivieillissement.

### DEFINITIONS

### « Conjugué peptidique/ brin peptidique »

Un « brin peptidique » est un polymère d'acides aminés, lesquels acides aminés sont reliés entre eux par au moins une liaison peptidique ou pseudopeptidique. Une « liaison peptidique » relie un groupement carbonyle CO et un groupement aminé NH dans un peptide. La liaison peptidique est donc une liaison amide reliant deux acides aminés entre eux. Ainsi les acides aminés sont dits « condensés » car ils ont perdu au moins une molécule d'eau pour former la ou les liaisons peptidiques dans lesquels ils sont impliqués. Un « conjugué peptidique » est un brin peptidique conjugué, i.e. lié, à un fragment moléculaire d'une autre nature, par exemple une chaîne hydrocarbonée en C₁₂ ou en C₁₆. Il est admis dans l'art que un peptide comprenant un nombre X d'acides aminé, il s'agit en réalité d'un nombre X d'acides aminés condensés, i.e. ayant chacun perdu une molécule d'eau (s'ils sont reliés les uns aux autres par une liaison peptidique, typiquement).

### « Liaison pseudo peptidique »

Ainsi, un peptide contient généralement entre 2 et environ quatre-vingt acides aminés, voire plus, la limite supérieure n'étant pas clairement définie. Un peptide comprenant deux résidus d'acides aminés est un dipeptide ; un peptide comprenant trois résidus d'acides aminés est un tripeptide ; un peptide comprenant deux résidus d'acides aminés est un tétrapeptide ; et ainsi de suite. L'expression « acide aminé » comprend toute molécule présentant au moins un acide carboxylique, au moins une amine et au moins un carbone reliant l'amine et l'acide carboxylique. Des acides aminés pouvant être utilisés dans le cadre de la présente invention sont tous types d'acides aminés, qu'ils soient naturels ou non, et en particulier ceux trouvés dans la séquence « SDKP ». Ainsi les acides aminés qui seront plus souvent retrouvés dans le cadre de la présente invention seront choisis parmi la sérine (« Ser », « S »), l'acide aspartique (« Asp », « D »), la lysine (« Lys », « K »), la proline (« Pro », « P ») leurs dérivés tels que leurs énantiomères, leurs diastéréoisomères, leurs isomères de positions, et leurs formes dites « protégées ».

Par formes dites « protégés » d'acides aminés, il est compris que les groupements fonctionnels de ces acides aminés sont substitués ou masqués par des groupements dits protecteurs adéquats choisis parmi ceux cités dans les ouvrages de référence Greene, "Protective Groups in Organic Synthesis", Wiley, New York, 2007 4e édition et/ou Harrison et al. "Compendium of Synthetic Organic Methods", Vol. 1 à 8 (J. Wiley & sons, 1971 to 1996).

Par « isomère de position », il est compris que certains des acides aminés tels que l'alanine peuvent se présenter avec des variations structurelles bien connues de l'homme du métier. Par exemple, l'alanine peut bien entendu se présenter sous sa forme alpha « a-Ala », mais également béta « (béta)-Ala » / « β-Ala ». A l'exception du cas de l'alanine qui comprends dans le cadre de la présente invention à la fois la forme alpha et la forme béta, les formes alpha d'acides aminés naturels sont toutefois privilégiées.

Dans le cadre de la présente invention les « acides aminés naturels » sont Lys, Ala, Gin, His. La glycine peut être considérée comme acide aminé naturel, mais elle ne comprend pas de carbone asymétrique.

Par « énantiomères » (ou « diastéréoisomères »), il est compris que les carbones asymétriques du squelette peptidique sont de configuration R ou S définissant ainsi des acides aminés (condensés) L ou D.

En outre, pour des questions de coûts de production liés à la pureté énantiomérique ou diastéréomérique des acides aminés engagés dans la synthèse peptidique, il peut être avantageux par exemple de synthétiser les peptides selon la présente invention avec un/des mélanges d'acides aminés de formes D et L. Le résultat de la synthèse est donc un mélange diastéréomérique de peptides selon la présente invention. Un objet de la présente invention concerne donc un mélange diastéréoisomérique de peptide ayant au moins un acide aminé condensé D/L en toutes proportions. Avantageusement le ou les acides aminés condensés est/sont racémique(s).

Par « mélange racémique », il est compris que le mélange d'énantiomères est d'environ 50/50 % en poids de chacun des énantiomères.

Le terme « environ » comprend des variations de ± 10% pour chacun des énantiomères dans le mélange. Ainsi, un « mélange racémique » couvre un mélange 40/60% en poids d'un mélange de deux énantiomères. N.B. : En toute rigueur, l'expression « mélange D/L » concerne les acides aminés avant leur incorporation dans les peptides. Toutefois, expérimentalement, l'utilisation d'un mélange racémique d'un acide aminé dans la synthèse d'un peptide de petite taille, comme c'est le cas selon la présente invention, permet d'obtenir un mélange d'environ 50/50% de diastéréoisomères du peptide synthétisé. Ainsi, par abus de langage, il est commun dans le domaine de faire référence à un « mélange racémique » d'un acide aminé condensé dans un peptide donné. Il est évident qu'il s'agit dans ce cas d'un mélange de diastéroisomères comme expliqué ci-dessus.

De manière avantageuse, les proportions d'acides aminés utilisés pour la synthèse des peptides selon la présente invention sont supérieurs ou égaux à 80%/20% en poids de chacun des énantiomères par rapport à la quantité totale de l'acide aminé (c'est-à-dire L et D) considéré.

Dans le cadre de la présente invention, pour considérer qu'un acide aminé est L ou D, il faut qu'il ait une proportion supérieure ou égale à 90%/10% en poids de chacun des énantiomères par rapport à la quantité totale de l'acide aminé (c'est-à-dire L et D) considéré.

Préférentiellement, les acides aminés « naturels » des peptides de formule (I) sont de forme L.

Les peptides relativement restreints en nombre d'acides aminés, comme ceux selon la présente invention, sont relativement faciles à synthétiser en phase liquide et/ou sur support solide. Ceci est un avantage indéniable de la présente invention.

Par « synthèse en phase liquide », il est compris les techniques de chimie organique en phase liquide à toutes échelles de grandeurs/volumes/poids confondues (c'est-à-dire des techniques adaptées à l'échelle du laboratoire de recherche - par exemple le milligramme, jusqu'aux quantités industrielles - par exemple la tonne). Par « synthèse sur support solide » il est compris les techniques de chimie organique sur support solide, et en particulier les techniques adaptées à la synthèse peptidique sur support solide.

Par exemple, des techniques de synthèse peptidique sont décrites dans l'ouvrage de Paul Lloyd-Williams, Fernando Albericio, Ernest Giralt, "Chemical Approaches to the Synthesis of Peptides and Proteins", CRC Press, 1997 ou Houben- Weyl, "Methods of Organic Chemistry, Synthesis of Peptides and Peptidomimetics", Vol E 22a, Vol E 22b, Vol E 22c, Vol E 22d., M. Goodmann Ed., Georg Thieme Verlag, 2002.

Typiquement, ces synthèses (tant en phase liquide que sur support solide) passent par une stratégie de « couplage-déprotection ». Cette stratégie consiste en l'activation de l'extrémité C-terminale (c'est-à-dire le COOH) d'un acide aminé/brin peptidique, de sa mise en présence avec un autre acide aminé ou brin peptidique, dont l'extrémité N-terminale (c'est-à-dire le NH₂) est libre. L'activation se fait par des techniques classiques de couplage par l'utilisation de DCC, de BOP, de PyBOP, d'anhydrides, d'anhydrides mixtes, chlorure d'acides, etc. Ces techniques de couplage peuvent être utilisées en variantes pour introduire des chaînes grasses reliées au reste de la molécule par l'intermédiaire de groupement CO ou oxygène.

Les acides aminés ou brin peptidiques engagés dans la réaction sont convenablement protégés afin de permettre ce couplage sélectivement entre les deux extrémités N et C-terminales voulues. Les fonctions pouvant réagir sont bien entendues masquées ou liées à des groupements protecteurs adéquats, tels que décrits ci-dessus.

L'étape de « couplage-déprotection » est répétée autant de fois que nécessaire avec les acides aminés/brins peptidiques voulus afin d'obtenir le peptide voulu. Le peptide final, s'il est supporté par une résine, est détaché. Les groupements protecteurs sont alors retirés. Les deux dernières étapes peuvent être faites en même temps dans le cas de synthèse sur support solide. De telles stratégies sont bien connues dans l'état de la technique. Par exemple, la protection du groupe amino de l'acide aminé peut être effectuée par un groupe tert-butyloxycarbonyle (désigné ci- après par Boc-) ou un groupe -9-fluorénylméthyloxycarbonyle (désigné ci-après par Fmoc-) représenté par la formule :

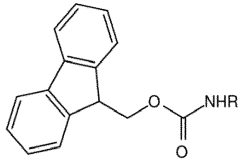

N.B. : « NHR » ci-dessus repésentant l'amine greffée par le Fmoc.

La protection est effectuée selon les procédés connus de l'art antérieur. Par exemple, la protection par le groupe Boc- peut être obtenue en faisant réagir l'acide aminé avec le di-tert-butylpyro-carbonate (Boc₂0).

Ces techniques sont très courantes dans le domaine, si bien qu'il est régulièrement fait mention de « stratégies Boc ou Fmoc » pour la synthèse des peptides.

Par exemple, les peptides selon la présente invention sont synthétisés par un enchaînement de cycles de déprotection/couplage, préférentiellement par la stratégie Fmoc/tBu.

L'avancement de chaque étape de déprotection/couplage est suivi par les techniques usuelles telles que la spectrométrie de masse.

Avantageusement, l'extrémité N-terminale libre du peptide n'ayant pas réagie dans la réaction de couplage, est acétylée à l'aide d'une solution d'anhydride acétique (étape dite de « capping »). L'étape finale de déprotection des chaînes latérales est effectuée préférentiellement en milieu acide, plus préférentiellement en présence d'acide trifluoroacétique (TFA).

Avantageusement, la synthèse s'effectue sur support solide, tel que sur des résines Rink amide, CTR (de type « chlorotrityle »), Wang, etc., et l'étape de déprotection finale acide permet en outre de détacher (« cliver ») le peptide de la résine.

Les techniques de purifications usuelles de peptides, tels que la précipitation, la cristallisation, la chromatographie liquide (en phase normale ou préférentiellement en phase inverse et à haute pression) sont appliquées aux peptides obtenus.

### « Peptide SDKP »

Le terme « SDKP » représente un brin peptidique de structure :

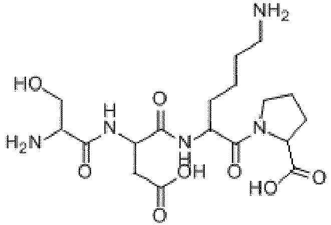

Avantageusement les acides aminés sont de configuration L :

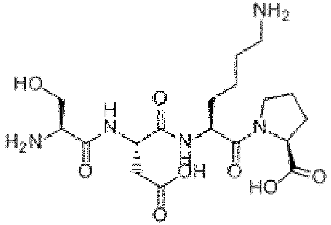

Les groupements fonctionnels (e.g. NH₂, OH, COOH) peuvent être sous forme protonée ou déprotonée selon le pH environnant. Ces groupements peuvent également être protégés par des groupements protecteur classiques tels que trouvés dans les ouvrages "Protective Groups in Organic Synthesis", Wiley, New York, 2007 4e édition et/ou Harrison et al. "Compendium of Synthetic Organic Methods", Vol. 1 à 8 (J. Wiley & sons, 1971 to 1996). Ainsi typiquement, les fonctions COOH peuvent être protégés par un groupement ester tel que OtBu, OMe, OEt, ou encore O-benzyle, la fonction OH par un éther de tBu, Me, Et, ou benzyle, et NH₂ par un groupement acido-labile tel que Boc ou baso-labile tel que Fmoc.

### « Analogue biologique »

Il est divulgué un « analogue biologique » est un composé présentant des propriétés biologiques similaires à la molécule initiale considérée, ici Ac-SDKP-OH. Ainsi, afin de déterminer si un composé est un analogue biologique de « Ac-SDKP-OH », il convient de savoir si les mêmes mécanismes d'actions physiologiques sont générés. De manière classique un test comparatif (entre la molécule d'intérêt et « Ac-SDKP-OH) *in vitro* permet de facilement vérifier ceci. Il est divulgué un test comparatif sur des cellules de peau humaine permet de savoir si le composé d'intérêt est un analogue biologique de « Ac-SDKP-OH ». De manière préférée, l'analogue biologique de « Ac-SDKP-OH » est un analogue biologique d'antivieillissement cutané (antirides ou restructurant de la peau). Ainsi, par « propriétés biologiques similaires », il est divulgué que la molécule d'intérêt présente une activité au moins égale à 90% de l'activité de Ac-SDKP-OH testée dans les mêmes conditions, en particulier sur les cellules de la peau. De manière typique, les analogues biologiques divulgués comprendront le brin peptidique de formule (II) suivante :

- Y₁-Y₂-Y₃-Y₄- (II)

dans lequel,
Y₁ représente l'extrémité N-terminale du fragment peptidique,
les fragments Y₃-Y₄ ou -Y₄ pouvant être absents du fragment peptidique de formule (II),
Y₁ représente la sérine ou un analogue structurel de la sérine,
Y₂ représente l'acide aspartique ou un analogue structurel de l'acide aspartique,
Y₃ représente la lysine ou un analogue structurel de la lysine,
Y₄ représente la proline ou un analogue structurel de la proline.

Par « analogue structurel de sérine », il peut être compris dans le contexte de la présente invention une sérine fonctionnalisée par un C₁-C₅ alkyle, C₁-C₅ alcène (telle que la N-méthyl-sérine), ou encore des molécules connues pour avoir des propriétés proches de la sérine telles que l'acide 4-amino-3-hydroxybutanoique, l'acide 2-amino-3-méthoxybutanoïque, ou encore l'O-benzyle-phosphosérine. Par « analogue structurel de l'acide aspartique », il peut être compris dans le contexte de la présente invention un acide aspartique fonctionnalisé par un C₁-C₅ alkyle, C₁-C₅ alcène (telle que la N-méthyl-acide aspartique), ou encore des molécules connues pour avoir des propriétés proches de l'acide aspartique, telles que l'acide glutamique, l'acide 2,6-diaminoheptanedioique, ou encore le benzoate de la chaîne latérale de l'acide aspartique (NH₂-Asp(OBn)-OH).

Par « analogue structurel de lysine», il peut être compris dans le contexte de la présente invention une lysine fonctionnalisée par un C₁-C₅ alkyle, C₁-C₅ alcène (telle que la N-méthyl-lysine, la lysine doublement méthylée (NH₂-Lys(Me₂)-OH), ou encore des molécules connues pour avoir des propriétés proches de la lysine telles que l'arginine, la lysine azide (NH₂-Lys(N₃)-OH), l'ornithine, l'acide 2-amino-3-guanidinopropionique, la citrulline, ou encore la lysine acétylée (NH₂-Lys(Ac)-OH). Par « analogue structurel de proline», il peut être compris dans le contexte de la présente invention une proline fonctionnalisée par un C₁-C₅ alkyle, C₁-C₅ alcène (telle que la N-méthyl-proline), ou encore des molécules connues pour avoir des propriétés proches de la proline telles que la trans-4-fluoro-proline, la trans-4-tert-butoxy-proline, l'acide 3-phénylpyrrolidine-2-carboxylique, l'acide (2S,3aS,7aS)-octahydro-1H-indole-2-carboxylique, l'acide 4-benzyl-pyrrolidine-2-carboxylique, l'acide 4-phényl-pyrrolidine-2-carboxylique, ou encore l'acide 4-hydroxypyrrolidine-2-carboxylique.

Il est divulgué un analogue biologique de nature peptidique, présentant avantageusement « SD », « SDK », ou « SDKP » dans sa séquence. De manière préférée, le brin « SD », « SDK », ou « SDKP » est compris dans les 10 derniers acides aminés de l'extrémité N-terminale de l'analogue biologique peptidique. De manière plus préférée, le brin « SD », « SDK », ou « SDKP » est compris dans les 5 derniers acides aminés de l'extrémité N-terminale de l'analogue biologique peptidique. De manière encore plus préférée, le brin « SD », « SDK », ou « SDKP » est à l'extrémité N-terminale de l'analogue biologique peptidique.

Avantageusement, l'analogue biologique de nature peptidique comprend moins de 20 acides aminés, plus avantageusement moins de 10 acides aminés, et encore plus avantageusement, il comprend moins de 6 acides aminés, ou moins de 5 acides aminés. Les analogues peptidiques de Ac-SDKP sont bien connus dans l'art et illustrés par exemple dans les articles Ma X et al. (Int J Cardiol. 2014 Aug 1;175(2):376-8), Zhuo JL et al (Am J Physiol Heart Circ Physiol. 2007 Feb;292(2), Gaudron S. et al. (Stem Cells, 1999,17, 2,100-106 ; J. Med. Chem., 1997,40, 24, 3963-3968) ou encore dans la demande de brevet internationale WO2004096292(et les références dont il est fait mention dans cette demande).

Par exemples des analogues identifiés dans l'art antérieur de Ac-SDKP-OH sont : N-acétyl-Ser(CH₂-NH)-Asp-Lys-Pro-OH ; N-acétyl-Ser-Asp(CH₂-NH)-Lys-Pro-OH ; N-acétyl-Ser-Asp-Lys(CH₂-NH)-Pro-OH ; N- acétyl-Ser-Asp-Lys-Pro-NH₂; N-acétyl-Ser-Asp-Lys-OH. Il est implicite dans cette divulgation que le fragment « Ac » dans ces molécules peut être remplacé par une chaîne grasse.

### « Chaîne grasse »

Par « chaîne grasse », selon la présente invention, il est compris toute chaîne linéaire ou ramifiée de nature organique ou (partiellement) inorganique hydrophobe, préférentiellement comprenant au moins 3 atomes (lorsque cette valeur n'est pas précisée) liés successivement les uns aux autres. Cette chaîne grasse peut être hydrocarbonée exclusivement, ou comprendre des hétéroatomes tels que O, S, Si, N, F, Cl, Br, I ou P.

De manière préférée, les chaînes grasses selon la présente invention sont des chaînes hydrocarbonées exclusivement, éventuellement insaturées, pouvant comprendre un ou plusieurs noyaux aromatiques (i.e. phényle).

Ainsi, la chaîne grasse peut être en C₁₃-C₅₀ linéaire ou ramifiée, saturée ou insaturée.

### « Alkyle substitué ou non substitué, linéaire ou ramifié en C₁₃-C₅₀ »

L'expression « alkyle substitué ou non substitué, linéaire ou ramifié en C₁₃-C₅₀», représente une chaîne hydrocarbonée saturée, linéaire ou ramifiée, comportant de 13 à 50 atomes de carbone, comme par exemple un groupement n-tridécyle (C13), n-tétradécyle (C14), n-pentadécyle (C15), n-hexadécyle (16), n-heptadécyle (C17), n-octadécyle (C18), n-nonadécyle (C19), n-eicosyle (C20), 1-méthyl-dodécyle (C13), 2-méthyl-dodécyle (C13), 1-éthyl-undécyle (C13), 2-éthyl-undécyle (C13), etc. Les alkyles peuvent être substitués ou non. Préférentiellement les alkyles peuvent être substitués par un ou plusieurs halogènes tels que F, Cl, Br, I, ou par d'autres fragments tels que OH, SH, NO₂, NH₂, COOH. Les alkyles peuvent en outre comprendre ou consister en une partie cyclique, tel qu'un motif cyclohexyl, ou cyclopentyl.

### « Aryle »

Par groupement « aryle », on entend un groupement aromatique, comportant de préférence de 5 à 18 atomes de carbone, comprenant un ou plusieurs cycles et comprenant éventuellement un ou plusieurs hétéroatome(s) en particulier un oxygène, un azote ou un soufre, comme par exemple un groupement phényle, furane, indole, pyridine, naphtalène, anthracène, etc. De préférence, par groupement « aryle », il est compris le groupement phényle.

### « Alkyle-aryle substitué ou non substitué, linéaire ou ramifié en C₂₁-C₅₀ »

Dans la présente invention, l'expression « Alkyle-aryle substitué ou non substitué, linéaire ou ramifié en C₂₁-C₅₀ » représente un fragment alkyle en C₁ à C₄₅ comme défini ci-dessus, dans lequel au moins un atome d'hydrogène a été remplacé par au moins un groupement aryle, dans lequel la somme totale des atomes de carbones présents dans l'alkyle-aryle est compris entre 21 et 50. Les alkyle-aryles selon la présente invention peuvent être substitués ou non. Préférentiellement les alkyle-aryles selon la présente invention peuvent être substitués par un ou plusieurs halogènes tels que F, Cl, Br, I, ou par d'autres fragments tels que OH, SH, NO₂, NH₂, COOH.

Par exemple, les alkyles en C₁ à C₄₅ substitués par un ou plusieurs aryles comprennent les groupements 1-phénylheptyle, 2-phénylheptile, 3-phénylheptyle, 4-phénylheptile, 5-phénylheptyle, 6-phénylheptile, 7-phénylheptyle, 1-phényloctyle, 2-phényloctyle, 3-phényloctyle, 4-phényloctyle, 5-phényloctyle, 6-phényloctyle, 7-phényloctyle, 8-phényloctyle, 1-phénylnonyle, 2-phénylnonyl, etc., 1-naphthyl-propyle, 2-naphthyl-propyle, 3-naphthyl-propyle, 1-anthracyl-methyle, etc.

De manière préférée, les alkyles en C₁ à C₄₅ substitués par un ou plusieurs aryles sont de alkyles en C₇-C₄₄ substitués par au moins un phényle. De manière encore plus préférée, les alkyles en C₁ à C₄₅ substitués par un ou plusieurs aryles sont de alkyles en C₁₂-C₄₄ substitués par au moins un phényle.

### « Alcènyle substitué ou non substitué, linéaire ou ramifié en C₁₃-C₅₀ »

Dans la présente invention, l'expression « alcènyle substitué ou non substitué, linéaire ou ramifié en C₁₃-C₅₀ » représente un fragment hydrocarboné comprenant au moins une insaturation, c'est-à-dire une double liaison covalente. Les alcènyles selon la peuvent être substitués ou non. Préférentiellement les alcènyles peuvent être substitués par un ou plusieurs halogènes tels que F, Cl, Br, I, ou par d'autres fragments tels que OH, SH, NO₂, NH₂, COOH.

Des exemples de tels alcényles sont un groupement n-1-tridécènyle (C13), n-2-tridécènyle (C13), n-3-tridécènyle (C13), n-4-tridécènyle (C13), n-5-tridécènyle (C13), n-6-tridécènyle (C13), n-7-tridécènyle (C13), n-8-tridécènyle (C13), n-9-tridécènyle (C13), n-10-tridécènyle (C13), n-11-tridécènyle (C13), n-12-tridécènyle (C13), n-1-tétradécènyle (C14), n-1-pentadécènyle (C15), n-1-hexadécènyle (16), n-1-heptadécènyle (C17), n-1-octadécènyle (C18), n-1-nonadécènyle (C19), n-1-eicosènyle (C20), etc.

Les alcènyles peuvent être substitués ou non. Préférentiellement les alcènyles peuvent être substitués par un ou plusieurs halogènes tels que F, Cl, Br, I, ou par d'autres fragments tels que OH, SH, NO₂, NH₂, COOH.

### « Guanidine » ou « guanidinium »

Un groupement guanidine est représenté par la formule :

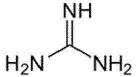

Ce groupement est typiquement relié à une autre molécule par l'un des atomes d'azote dans la représentation ci-dessus. La protonation de cette guanidine forme un cation stabilisé par résonance. La charge positive, dans ce cas permet une lipophilicité à ce groupement. C'est typiquement le cas des argines protonées par exemples, utilisées afin d'augmenter la lipophilicité d'un brin peptidique.

### « Sels pharmaceutiquement acceptables »

Selon la présente invention, l'expression « sels pharmaceutiquement acceptables » concerne un sel d'addition d'acide formé de préférence par des acides libres pharmaceutiquement acceptables. Un sel d'addition d'acide peut être obtenu à partir d'acide(s) inorganique(s) comme l'acide chlorhydrique, l'acide nitrique, l'acide phosphorique, l'acide sulfurique, l'acide bromhydrique, l'acide iodhydrique, l'acide nitreux et l'acide phosphoreux, ou à partir d'acide(s) organique(s) non toxique(s) tels qu'un composés aliphatique mono / dicarboxylates, un groupe phényle alcanoate substitué, hydroxy alcanoate, alkandioate, les acides aromatiques et aliphatiques / aromatiques des acides sulfoniques.

Les sels non toxiques pharmaceutiquement peuvent être un sulfate, un pyrosulfate, un bisulfate, un sulfite, un bisulfite, un nitrate, un phosphate, un monohydrogénophosphate, un dihydrogénophosphate, un métaphosphate, un pyrophosphate, un chlorure, un bromure, un iodure, un fluorure, un acétate, un propionate, un décanoate, un caprylate, un acrylate , un formiate, un isobutylate, un caprate, un heptanoate, un propiolate, un oxalate, un malonate, un succinate, un fumarate, un benzoate, un chlorobenzoate, un méthylbenzoate, un dinitrobenzoate, un hydroxybenzoate, un méthoxybenzoate, un phtalate, un téréphtalate, un benzènesulfonate, un toluènesulfonate, un chlorobenzène, un xylènesulfonate, un phénylacétate, un phénylpropionate, phenylbutylate, un citrate, un lactate, un hydroxybutylate, un glycolate, un malate, un tartrate, un méthanesulfonate, un propanesulfonate, un naphtalène-1-sulfonate, un naphtalène-2-sulfonate et un mandélate.

Les sels d'additions acides de la présente invention peuvent être préparés par les procédés classiques connus de l'homme du métier. Par exemple, les composés de la présente invention sont dissous dans une solution aqueuse acide. Ensuite, le sel est obtenu par précipitation de la solution en utilisant un solvant organique miscible à l'eau tel que le méthanol, l'éthanol, l'acétone ou l'acétonitrile. Les sels peuvent être obtenus par tout autre moyen connu.

Des sels pharmaceutiquement acceptables métalliques peuvent être préparés en utilisant une ou plusieurs bases. Le métal alcalin ou le sel de métal alcalin peut être obtenu par les procédés suivants: dissolution du composé dans un excès d'hydroxyde de métal alcalin ou dans une solution d'hydroxyde de métal alcalino-terreux; filtration du sel de composé non soluble; évaporation de la solution et séchage de celui-ci. A ce moment, le sel de métal est de préférence préparé dans une forme pharmaceutiquement appropriée tel que qu'un sel de sodium, potassium ou sel de calcium.

### « Néogoutte »/ « nanoparticule lipidique »

Dans le cadre de la présente invention, les peptides actifs peuvent être insérés dans une nanoparticule telle que divulgué dans FR2991196. Cette nanoparticule comprend plusieurs phases lipidiques/aqueuses. De par ses propriétés particulières trouvées avec les peptides de l'invention, la Demanderesse a attribué le nom de « néogoutte » à ces nanoparticules. Néanmoins, selon la présente invention, le terme « néogoutte » est synonyme de « nanoparticule lipidique » et ces expressions sont donc équivalentes.

### « Phase lipidique »

Par « phase lipidique » selon la présente invention, il est compris toute phase présentant une affinité avec les solvants organiques et les lipides (huiles ou cires) et évitant d'être en contact avec un solvant polaire, comme l'eau. Il peut s'agir des corps gras tels que définis dans FR2934955 et FR2991196. Par exemple, la phase lipidique peut comprendre des phospholipidiques tels que définis dans en page 7, lignes 7 à 13 de FR2934955, de la lécithine telle que définie en page 7, lignes 14 à 19 de FR2934955, des acides gras tels que définis en page 7, lignes 20 à 25 de FR2934955, les lipides amphiphiles tels que divulgué de la page 13, ligne 8 à la page 14 ligne 30 de FR2991196.

### « Agent tensioactif »

Dans le cadre de la présente invention, l'expression « agent tensioactif » représente une molécule amphiphile présentant deux parties de polarités différentes, l'un lipophile (apolaire) et l'autre hydrophile (polaire). L'agent tensioactif peut être ionique, non-ionique ou zwiterrionique (cationique et anionique). Par exemple, les tensioactifs selon la présente invention peuvent être choisis dans ceux divulgués dans FR2991196 et/ou FR2934955, tels que ceux trouvés de la page 4, ligne 28 à page 6, ligne 3 de FR2991196, ou page 11, ligne 19 à page 12 ligne 11 de FR 2934955.

### « Voie topique »

Par « voie topique », il est compris l'administration des peptides et/ou des compositions contenant les peptides directement sur les zones de peau à traiter.

### « Utilisation antivieillissement »

L'objet de la présente invention a notamment pour but une utilisation antivieillissement, i.e. le vieillissement intrinsèque de la peau.

Par « vieillissement intrinsèque de la peau », il est compris qu'il s'agit d'une altération naturelle de la peau en fonction du temps, se caractérisant par l'apparition de rides, ridules, crevasses, etc.

Il est divulgué un kit antivieillissement. Par « kit antivieillissement » il est compris un dispositif comprenant un ensemble d'éléments dont au moins un élément est destiné à réparer le vieillissement intrinsèque de la peau et dont au moins un élément comprend ou consiste en un peptide selon l'invention. Préférentiellement ledit peptide est compris dans une composition selon l'invention.

### DESCRIPTION DETAILLEE

L'objet de la présente invention concerne plus particulièrement un conjugué peptidique tel que décrit ci-dessus, caractérisée en que ledit conjugué est de formule (I) suivante :

R₁-X₁-X₂-X₃-X₄₋A₁-R₂ (I)

dans laquelle :
- R₁, étant du côté N-terminal, est un atome d'hydrogène, ou une chaîne grasse choisie parmi un groupe alkyle substitué ou non substitué, linéaire ou ramifié en C₁₃-C₅₀, un groupe alkyle-aryle substitué ou non substitué, linéaire ou ramifié en C₂₁-C₅₀, un groupe alcényle substitué ou non substitué, linéaire ou ramifié en C₁₃-C₅₀, un groupe R₃-CO-, R₃-OCO- ou R₃-COO- dans lequel R₃ est un groupe alkyle ou alcènyle substitué ou non substitué, linéaire ou ramifié en C₁₃-C₄₉, les substitutions incluant F, Cl, ou tout hétéroatome ou groupe d'hétéroatomes lipophile tel qu'une guanidine ou un guanidinium,
- X₁ est un condensat de (L)-sérine,
- X₂ est un condensat de (L) et/ou (D) acide aspartique,
- X₃ est un condensat de (L) et/ou (D) lysine, ,
- X₄ est un condensat de (L) et/ou (D) proline,
- X₄ étant optionnellement absent,
- les liaisons reliant X₁ à X₂, X₂ à X₃ le cas échéant, et X₃ à X₄ le cas échéant, pouvant être des liaisons peptidiques ou pseudopeptidiques,
- A₁ est une liaison covalente, un groupement NH ou un atome d'oxygène,
- R₂ est un atome d'hydrogène, ou une chaîne grasse choisie parmi un groupe alkyle substitué ou non substitué, linéaire ou ramifié en C₁₃-C₅₀, un groupe alkyle-aryle substitué ou non substitué, linéaire ou ramifié en C₂₁-C₅₀, un groupe alcènyle substitué ou non substitué, linéaire ou ramifié en C₁₃-C₅₀, un groupe R₄-CO-, ou R₄-OCO- dans lequel R₄ est un groupe alkyle ou alcènyle substitué ou non substitué, linéaire ou ramifié en C₁₃-C₅₀, les substitutions incluant F, Cl, ou tout hétéroatome ou groupe d'hétéroatomes lipophile tel qu'un groupement guanidine ou guanidinium,
- R₁ et R₂ ne pouvant être conjointement des atomes d'hydrogène,
ou l'un de ses sels pharmaceutiquement acceptables.

Par « condensat », il est compris dans le contexte de la présente invention la partie restante de l'acide aminé suite à la formation de la liaison peptidique entre deux acides aminés (dans le cas d'une réaction entre deux acides aminés), une molécule d'eau ayant été éliminée. Par exemple dans le cas de la sérine :

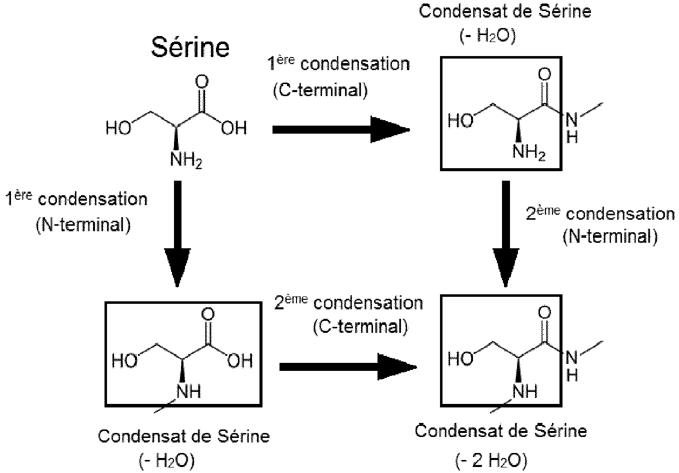

Dans la figure ci-dessus, à chaque étape de condensation, l'élimination de H₂O implique que OH ou H est fourni par la sérine. Néanmoins, même si l'acide aminé n'est pas condensé sur ses deux extrémités (N-terminale et C-terminale), par abus de langage, il est considéré qu'il s'agit d'un condensat. Ceci explique d'ailleurs pourquoi en chimie peptidique, la nomenclature s'accorde à ajouter un H en N-terminal ou un OH en C-terminal, le cas échéant : H-AA₁-AA₂-... AAₙ-OH. Tout ceci fait partie des connaissances générales de l'homme du métier.

L'objet de la présente invention concerne également une néogoutte (nanoparticule lipidique) selon la présente invention caractérisée en ce que le conjugué peptidique est tel que décrit présentement. L'objet de la présente invention concerne également une néogoutte (nanoparticule lipidique) selon la présente invention caractérisée en ce qu'elle comprend en outre le conjugué peptidique de formule Ac-SDKP-OH, Ac-SDKP-NH₂ ou un mélange des deux.

L'objet de la présente invention concerne également une néogoutte telle que définie ci-dessus caractérisée en ce que le conjugué peptidique couplé à cette néogoutte est de formule (I) telle que définie ci-dessus, dans laquelle :
- R₁, étant du côté N-terminal, est un atome d'hydrogène, un groupe alkyle substitué ou non substitué, linéaire ou ramifié en C₃-C₅₀, un groupe alkyle-aryle substitué ou non substitué, linéaire ou ramifié en C₈-C₅₀, un groupe alcènyle substitué ou non substitué, linéaire ou ramifié en C₃-C₅₀, un groupe R₃-CO-, R₃-OCO- ou R₃-COO- dans lequel R₃ est un groupe alkyle ou alcènyle substitué ou non substitué, linéaire ou ramifié en C₃-C₅₀, les substitutions incluant F, Cl, ou tout hétéroatome ou groupe d'hétéroatomes lipophile tel qu'un groupement guanidine ou guanidinium, et
- R₂ est un atome d'hydrogène, un groupe alkyle substitué ou non substitué, linéaire ou ramifié en C₃-C₅₀, un groupe alkyle-aryle substitué ou non substitué, linéaire ou ramifié en C₈-C₅₀, un groupe alcènyle substitué ou non substitué, linéaire ou ramifié en C₃-C₅₀, un groupe R₄-CO-, ou R₄-OCO- dans lequel R₄ est un groupe alkyle ou alcènyle substitué ou non substitué, linéaire ou ramifié en C₃-C₅₀, les substitutions incluant F, Cl, ou tout hétéroatome ou groupe d'hétéroatomes lipophile tel qu'un groupement guanidine ou guanidinium.

De manière préférée, l'objet de la présente invention concerne en outre une néogoutte telle que définie ci-dessus caractérisée en ce que R₁ est un groupe alkyle substitué ou non substitué, linéaire ou ramifié en C₆-C₃₀, préférentiellement en C₁₀-C₂₀, ou un groupe R₃-CO- dans lequel R₃ est un groupe alkyle ou alcènyle substitué ou non substitué, linéaire ou ramifié en C₅-C₃₀, préférentiellement en C₉-C₂₀, les substitutions incluant F, Cl, ou tout hétéroatome ou groupe d'hétéroatomes lipophile tel qu'un groupement guanidine ou guanidinium.

De manière plus préférée, l'objet de la présente invention concerne une néogoutte telle que définie ci-dessus caractérisée en ce que R₁ est un groupe alkyle substitué ou non substitué, linéaire ou ramifié en C₁₂-C₁₈, préférentiellement en C₁₄-C₁₆, ou un groupe R₃-CO- dans lequel R₃ est un groupe alkyle ou alcènyle substitué ou non substitué, linéaire ou ramifié en C₁₀-C₁₈, préférentiellement en C₁₃-C₁₆, les substitutions incluant F, Cl, ou tout hétéroatome ou groupe d'hétéroatomes lipophile tel qu'un groupement guanidine ou guanidinium.

De manière encore plus préférée, l'objet de la présente invention concerne une néogoutte telle que définie ci-dessus caractérisée en ce que R₁ est un groupe alkyle substitué ou non substitué, linéaire ou ramifié en C₁₂, C₁₃, C₁₄, C₁₅, C₁₆, C₁₇, C₁₈, C₁₉ ou C₂₀ ou un groupe R₃-CO- dans lequel R₃ est un groupe alkyle ou alcènyle substitué ou non substitué, linéaire ou ramifié en C₁₂, C₁₃, C₁₄, C₁₅, C₁₆, C₁₇, C₁₈, C₁₉ ou C₂₀ les substitutions incluant F, Cl, ou tout hétéroatome ou groupe d'hétéroatomes lipophile tel qu'un groupement guanidine ou guanidinium.

En outre, l'objet de la présente invention concerne des néogouttes telle que définies ci-dessus, comprenant un conjugué peptidique selon la présente invention, préférentiellement de formule (I), caractérisé en ce que le peptide est présent en surface de la néogoutte.

De manière typique, lors de la fabrication des néogouttes selon la présente invention, le conjugué peptidique (I) se réparti entre la phase aqueuse libre et la couche superficielle desdites néogouttes. En d'autres termes, une portion du conjugué peptidique (I) s'attache à la néogoutte par le biais de sa chaîne grasse et l'autre portion de conjugué peptidique (I) reste libre dans la phase aqueuse. Cette répartition du conjugué peptidique selon la présente invention varie selon la nature de la chaîne grasse sur ledit conjugué peptidique : plus la chaîne grasse est grande, naturellement, plus la proportion de conjugué peptidique lié à la néogoutte est grande. Cette répartition du conjugué peptidique varie également, mais dans une moindre mesure selon la nature de la phase L₁ et/ou L₂. Ainsi, s'il faut au minimum que la chaîne grasse du conjugué peptide (I) comprenne 3 atomes de carbone pour commencer à constater des néogouttes liées avec des conjugués peptidique (I), un conjugué peptidique (I) comprenant une chaîne grasse de 10 à 15 atomes de carbone induira de manière typique un taux de liaison aux néogouttes compris entre 20 et 50% en poids. Par exemple un conjugué peptidique (I) comprenant une chaîne grasse de 12 atomes de carbone induira typiquement un taux de liaison aux néogouttes d'environ 25 à 30% en poids dans les conditions classiques selon la présente invention. Ainsi, de manière générale, la néogoutte telle que définie ci-dessus est caractérisée en ce qu'elle comprend entre 5 et 95% en poids de conjugué peptidique (I) lié à la néogoutte, avantageusement entre 10 et 80% en poids, plus avantageusement entre 20 et 60% en poids, encore plus avantageusement entre 25 et 50% en poids vis-à-vis de la quantité totale de conjugué peptidique (I).

De manière avantageuse, les néogouttes selon la présente invention sont diluées dans un solvant aqueux, préférentiellement comprenant au moins 50% d'eau en poids/poids total, plus préférentiellement au moins 75% d'eau, encore plus préférentiellement au moins 90% d'eau, tel qu'au moins 95%, au moins 97% ou au moins 99% d'eau. De manière préférée, cette dilution est d'au moins 1 : 1 en poids (poids néogouttes : poids solvant aqueux), préférentiellement au moins 1 : 5 (poids néogouttes : poids solvant aqueux), plus préférentiellement au moins 1 : 7 (poids néogouttes : poids solvant aqueux). De manière toute préférée, cette dilution est comprise entre 1 : 5 en poids (poids néogouttes : poids solvant aqueux) et 1 : 100 (poids néogouttes : poids solvant aqueux), avanatageusement comprise entre 1 : 5 en poids (poids néogouttes : poids solvant aqueux) et 1 : 50 (poids néogouttes : poids solvant aqueux), telle que environ 1 : 10 (poids néogouttes : poids solvant aqueux).

Ainsi, l'objet de la présente invention concerne une néogoutte telle que définie ci-dessus caractérisée en ce que ladite néogoutte comprend au moins l'un des constituants suivants:
- au moins un acide gras en C₅-C₃₀, éventuellement hydrogéné et/ou sous forme d'ester de glycol, dans la phase (L₁),
- un ester d'acide gras en C₁-C₃₀ de polyoxyéthylène (10 - 100) comme agent tensioactif,
- au moins un acide gras en C₅-C₃₀, éventuellement hydrogéné et/ou sous forme d'ester de glycol, dans la phase (L₂), et/ou
- au moins un conservateur par exemple de type phénoxyéthanol dans la phase (A₁).

Préférentiellement, l'objet de la présente invention concerne une néogoutte telle que définie ci-dessus caractérisée en ce que ladite néogoutte comprend au moins l'un des constituants suivants:
- au moins un acide gras en C₁₀-C₂₀, éventuellement hydrogéné et/ou sous forme d'ester de glycol, dans la phase (L₁),
- un ester d'acide gras en C₁₀-C₂₀ de polyoxyéthylène (10 - 100) comme agent tensioactif,
- au moins un acide gras en C₁₀-C₂₀, éventuellement hydrogéné et/ou sous forme d'ester de glycol, dans la phase (L₂), et/ou
- au moins un conservateur par exemple de type phénoxyéthanol dans la phase (A₁).

Plus préférentiellement, l'objet de la présente invention concerne une néogoutte telle que définie ci-dessus caractérisée en ce que ladite néogoutte comprend au moins l'un des constituants suivants:
- au moins un acide gras en C₁₂, C₁₃, C₁₄, C₁₅, C₁₆, C₁₇, C₁₈, C₁₉ ou C₂₀, éventuellement hydrogéné et/ou sous forme d'ester de glycol, dans la phase (L₁),
- un ester d'acide gras en C₁₂, C₁₃, C₁₄, C₁₅, C₁₆, C₁₇, C₁₈, C₁₉ ou C₂₀ de polyoxyéthylène (10 - 100) comme agent tensioactif,
- au moins un acide gras en C₁₂, C₁₃, C₁₄, C₁₅, C₁₆, C₁₇, C₁₈, C₁₉ ou C₂₀, éventuellement hydrogéné et/ou sous forme d'ester de glycol, dans la phase (L₂), et/ou
- au moins un conservateur par exemple de type phénoxyéthanol dans la phase (A₁).

Plus précisément, l'objet de la présente invention concerne une néogoutte telle que définie ci-dessus caractérisée en ce que le conjugué peptidique comprend la séquence (L)S-(L)D-(L)K-(L)P.

L'objet de la présente invention peut concerner une néogoutte telle que définie ci-dessus caractérisée en ce que le conjugué peptidique comprend la séquence (L)S-(L)D-(L)K.

L'objet de la présente invention concerne une néogoutte telle que définie ci-dessus caractérisée en ce que le conjugué peptidique comprend la séquence (L)S-(L)D.

L'objet de la présente invention concerne en outre un procédé de fabrication de la néogoutte selon la présente invention. Le procédé est proche de celui décrit dans FR2991196 (cf. page 17, ligne 26 à page 19, ligne 27, à ceci près que le conjugué peptidique (principe actif) selon la présente invention est greffé sur la partie extérieure et non le cœur de la néogoutte. Ceci peut se faire en ajoutant le conjugué peptidique lors de la formation de la néogoutte ou après. De manière préférée de par les affinités du conjugué peptidique selon la présente invention, celui-ci va se positionner de manière naturelle en surface de la néogoutte. Toutefois, il également possible d'additionner ce conjugué peptidique précisément au moment de la formation de la membrane interne L₂ par les techniques décrites dans FR2991196,

Ainsi, l'objet de la présente invention concerne une composition cosmétique comprenant un conjugué peptidique selon la présente invention à une teneur comprise entre 1.10⁻⁹% et 10% en poids relatif à la quantité totale de ladite composition. Préférentiellement, le conjugué peptidique selon la présente invention est compris dans ladite composition cosmétique dans une néogoutte selon la présente invention. De manière avantageuse, la composition cosmétique selon la présente invention contient le conjugué peptidique à une teneur comprise entre 1.10⁻⁷% et 1% en poids, plus avantageusement à une teneur comprise entre 1.10⁻⁶% et 0.1% en poids, voire à une teneur comprise entre 2.10⁻⁶% et 0.01% en poids. De manière toute préférée, la teneur en conjugué peptidique selon la présente invention est d'environ 5.10⁻⁶% en poids.

Un mode de réalisation particulier selon la présente invention concerne une composition cosmétique comprenant un conjugué peptidique de formule (I), avec en outre Ac-SDKP-OH et/ou Ac-SDKP-NH₂. De manière préférée, Ac-SDKP-OH et/ou Ac-SDKP-NH₂ est compris au moins partiellement dans la néogoutte selon la présente invention.

La composition cosmétique selon la présente invention peut être conditionnée dans tout moyen apte à son application, tel qu'un pot, un tube, un flacon, un spray, une boite.

La composition cosmétique selon la présente invention peut être administrée de manière topique une ou plusieurs fois par jour, préférentiellement au moins une fois le matin et une fois le soir.

La composition cosmétique peut se présenter sous forme d'une crème, d'un gel, d'une lotion, d'un sérum ou encore d'une pâte.

### FIGURES

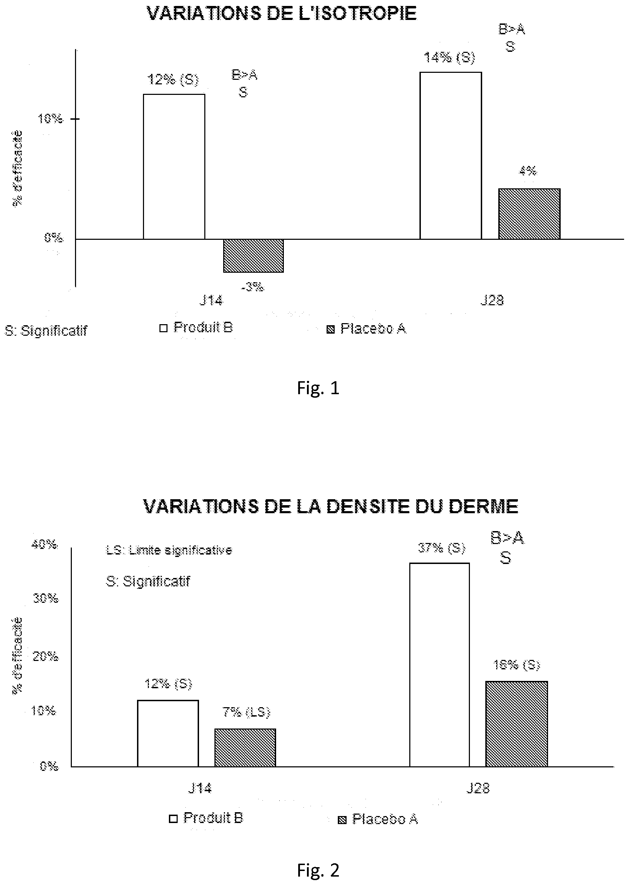

L'histogramme de la figure 1 représente les variations de l'isotropie suite à l'étude comparative selon l'exemple 4 ci-dessous. Le produit B comprend du Lauroyl-SDKP-OH, tandis que le placebo A est une formulation de néogoutte exempt d'analogues de SDKP. Il peut être vu l'effet significatif sur le relief de la peau (isotropie - orientation des lignes de la peau) du produit B vis-à-vis du placebo. L'histogramme de la figure 2 représente les variations de la densité du derme suite à l'étude comparative selon l'exemple 4 ci-dessous. Le produit B comprend du Lauroyl-SDKP-OH, tandis que le placebo A est une formulation de néogoutte exempt d'analogues de SDKP. Il peut être vu l'effet significatif sur la densité du derme du produit B vis-à-vis du placebo.

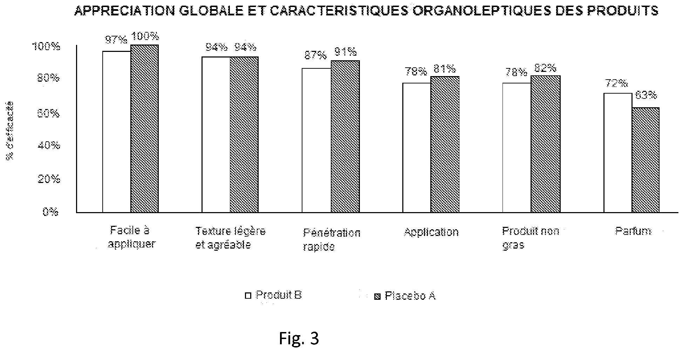

L'histogramme de la figure 3 représente l'appréciation globale et caractéristiques organoleptiques des produits (facilité d'application, texture légère et agréable, pénétration rapide, application, produit non gras, parfum). Il peut être vu que les formulations testées présentent des caractéristiques organoleptiques et des appréciations générales similaires et acceptables.

### EXEMPLES

### Exemple 1 : Synthèses du composé « lauroyl-SDKP-OH »

La synthèse décrite ci-dessous est à but d'illustration. Elle peut par exemple s'effectuer sur support solide (résine) en stratégie Fmoc (sur robot pour des quantités inférieure à 50 mg et en manuel pour des quantités supérieures). Les taux de charges des résines et les concentrations des réactifs (agents de couplage, bases, acides, agents de piégeage (« scavengers » en anglais)) peuvent varier selon la pratique courante dans l'art.
- Les matières premières utilisées peuvent être:
   - des acides aminés naturels protégés sur leurs extrémités N-terminale (protection Fmoc) et sur leurs chaines latérales (groupements protecteurs acido-labiles et donc compatibles avec la stratégie Fmoc).
   - des activateurs (HOBT/DIC). Ces produits sont éliminés par lavage tout au long de la synthèse, puis lors de la phase de précipitation et enfin lors de la phase de purification.
   - des solvants de lavage (DMF, DCM). Ces produits sont éliminés par lavage tout au long de la synthèse, puis lors de la phase de précipitation et enfin lors de la phase de purification.
   - résine Wang préchargée avec l'acide aminé Fmoc-L-Pro. Le taux de charge initial, ou réduit, de la résine en proline peut varier mais est typiquement compris entre 0,1 et 2,0 mol/g de résine, préférentiellement entre 0,5 et 1,5 mol/g, plus préférentiellement d'environ 0,75 mol/g ± 10%. La résine est éliminé par simple filtration à l'issu de la synthèse.
   - les bases usuelles pour retirer Fmoc sont utilisées telles que la triéthyle amine, DBU, pipéridine, etc. La pipéridine a été en particulier utilisée ici.
   - les acides usuels pour retirer le peptide du support ont été utilisés (e.g. TFA, HCl, Acide acétique, etc.). Dans le cas des résine « Wang », du TFA (acide trifluoroacétique) avec un piégeur (« scavenger »), tels que des dérivés de silicium usuels dans l'art (e.g. TIS).
- Le déroulé de la synthèse est le suivant:
   - Point de départ : résine préchargée avec la Fmoc-Proline ;
      1. Greffage du second acide aminé, lysine:
         - Déprotection de la proline supportée avec de la pipéridine (par exemple 20% dans du DMF) afin de retirer le groupement Fmoc ;
         - 3 séries de lavages au DMF ;
         - Couplage de Fmoc-L-Lys(Boc)-OH sur le peptide en élongation par l'utilisation de HOBt, DCI ;
         - 3 séries de lavages au DMF ;
         - Acétylation par l'utilisation d'anhydride acétique ou le chlorure d'acyle équivalent afin de bloquer les éventuelles amines de Pro n'ayant pas réagies.
         - 3 séries de lavages au DMF ;
      2. Greffage de l'acide aspartique Fmoc-L-Asp(OtBu)-OH :
         Les mêmes étapes que pour le greffage de la lysine sont reproduites pour l'acide aspartique.
      3. Greffage de la sérine Fmoc-L-Ser(tbu)-OH:
         Les mêmes étapes que pour le greffage de la lysine/acide aspartiques sont reproduites pour la sérine.
      4. Addition du fragment lauroyl sur l'amine de la sérine
         - Déprotection de la sérine avec de la pipéridine (20% dans du DMF) afin de retirer le groupement Fmoc ;
         - 3 séries de lavages au DMF ;
         - ajout de chlorure de lauroyle en présence de pyridine, ajout suivi par test à la ninhydrine (classique dans l'art) jusqu'à conversion complète ;
         - 3 séries de lavages au DMF ;
      5. clivage du peptide de la résine et déprotection
         - traitement de la résine par un mélange de TFA/TIS/H2O, 95/2.5/2.5
         - 3 précipitations à l'éther, centrifugation.

### Mise en solution d'une partie du culot pour analyse

### • Déroulé de l'analyse post synthèse:

L'analyse s'effectue sur une UPLC ACQUITY H Class WATERS et sur colonne BEH C18, 150x2.1mm
- **0 à 100% d'acétonitrile en 8.5 min,** puis 100% à 0% d'acétonitrile en 2 min, débit : 0.6 ml/min

L'analyse en spectrométrie de masse s'effectue sur Système LC/MS WATERS composé de 2695 Séparation Module Alliance; Colonne X-Bridge C18, 3,5µm, 4,6x150 mm ; 2996 Photodiode Array Detector ; SQ Mass Detector 2 (Analyseur : quadripôle et Source : ES+), débit : 0.6 ml/min:
- **3 à 97% d'acétonitrile en 15 min,**
- puis un plateau à 97% d'acétonitrile pendant 2 min,
- puis 97% à 3% d'acétonitrile en 2 min,
puis un plateau à 3% d'acétonitrile pendant 2 min.

### • Déroulé de la purification:

la purification s'effectue sur une HPLC préparative de marque waters LC4000 et sur colonne Vydac denali 50 x 300 mm, 10 micron dans un mélange H₂O(0.1% TFA)/Acétonitrile (0.1% TFA)

### • Déroulé de l'analyse post purification:

Analyse des fractions collectées sur HPLC selon le protocole décrit ci-dessus Les fractions correspondantes au cahier des charges sont regroupées, lyophilisées, puis ré-analysées en HPLC et en spectrométrie de masse selon les protocoles décrits ci-dessus.

Le produit final est essentiellement constitué du peptide Lauroyl-SDKP-OH, à plus de 90%.

Les impuretés présentes sont des peptides de délétion tels que Ac-P-OH, Ac-KP-OH, Ac-DKP-OH.

Les solvants, activateurs, protections,... sont éliminés au fur et à mesure de la synthèse, lors de la précipitation à l'éther (volatile) puis lors de la purification et enfin de la lyophilisation.

### Exemple 2 : Préparation de néogoutte

Une néogoutte selon la présente invention peut être préparée selon le mode opératoire divulgué dans FR2991196 sur la base des quantités selon le tableau 1 suivant :

**Tableau 1 : Composés impliqués dans la préparation de la néogoutte :**

| | Composé | Nom commercial | Fournisseur | Masse (mg) | % masse dans le procédé |
|---|---|---|---|---|---|
| Phase aqueuse | Eau | - | - | 1500 | 75,00 |
| | Stéarate de PEG 40 | Myrj s40 | Croda | 215 | 10,75 |
| Phase lipidique | Phospholipides | Phospholipon | Lipoid | 45 | 2,25 |
| | Huile d'olive | | | 115 | 5,75 |
| | Cire | Lipocire | Gattefossé | 115 | 5,75 |
| | Palmitoyl-KTTKS | - | Creative | 9,99 | 0,4995 |
| | | | Peptide | | |
| | Lauroyl-SDKP-OH Solution 1% | | Gen Pep | 0,01 | 0,0005 |

| | | | | | |
|---|---|---|---|---|---|
| N.B. : Les mêmes composés que ceux décrits dans FR2991196 ont été utilisés dans le cadre de la présente invention. | | | | | |

Il a pu être également constaté des possibilités de modifications de cette formulation lors du procédé de fabrication des néogouttes décrite ci-dessous:
- la quantité d'eau peut être comprise entre de 60 à 90% en masse par rapport à la masse totale des composés impliqués, par exemple 77,2% ;
- la quantité de stéarate de PEG 40 peut être comprise entre de 5 à 20% en masse par rapport à la masse totale des composés impliqués, par exemple 9,2% ;
- la quantité de phospholipides peut être comprise entre 1,5 et 3% en masse par rapport à la masse totale des composés impliqués, par exemple 1,75% ;
- la quantité de huile d'olive peut être comprise entre 3 et 10% en masse par rapport à la masse totale des composés impliqués, par exemple 4,49% ;
- la quantité de cire peut être comprise entre 3 et 10% en masse par rapport à la masse totale des composés impliqués, préférentiellement de même masse que l'huile d'olive, par exemple 4,49% ;
- la quantité de Palmitoyl-KTTKS peut par exemple être comprise entre 0,01 et 10% en masse par rapport à la masse totale des composés impliqués, par exemple 0,05% ;
- la quantité de Lauroyl-SDKP-OH peut par exemple être comprise entre 0,00001 et 1% en masse par rapport à la masse totale des composés impliqués, par exemple 0,00006%.

### A. Préparation de la phase aqueuse

La phase aqueuse a été préparée en solubilisant le tensioactif Myrj S40, dissous dans du tampon phosphate salin (PBS) 1X, dans l'eau.

### B. Préparation de la phase lipidique 30

La phase lipidique a été préparée en mélangeant de l'huile de soja (Soybean oil, Sigma Aldrich), de la paraffine (Semi-synthetic glycerides, Suppocire NC, Gattefossé, France), des phospholipides de graine de soja (Phospholipon 75, Lipoid, Allemagne) et 0.1% en masse de fluorophore Dil (1,1'-Dioctadecyl-3,3,3',3'-tetramethylindocarbocyanine perchlorate, Sigma Aldrich). La phase lipidique ainsi préparée contient 16% en masse de phospholipides et 84% en masse de lipides.

### C. Préparation des néogouttes par ultrasonication

20% de la phase lipidique ont été dispersés dans 80% de la phase aqueuse, conduisant à un mélange possédant un ratio phospholipides/Myrj S40 de 0,18 et un ratio Myrj S40/(huile + cire) de 0,55. L'émulsification du mélange a ensuite été réalisée avec une sonde ultrasonique de 3 mm, suivant des cycles de sonication (10s ON / 30s OFF) pendant 10 min.

Les suspensions de nanoémulsions peuvent ensuite être dialysées contre 500 mL de PBS 1X pendant une nuit. Puis, elles ont été récupérées, diluées à une teneur de 10% en masse, filtrées à travers des pores de 0,2 pm et enfin conservées à 4°C jusqu'à leur utilisation.

### Exemple 3 : Dilution

Il est possible de diluer les néogouttes selon la présente invention.

Un solvant hydrophile peut être utilisé à cette fin. Typiquement de l'eau est utilisée. Il est aussi possible d'ajouter des conservateurs, des antimicrobiens, ou tout autre composé (e.g. excipient) communément utilisé dans les formulations cosmétiques, dermo-cosmétiques et/ou pharmaceutiques.

D'un point de vue pratique, la dilution des néogouttes s'effectue simplement en additionnant un solvant de dilution (e.g. l'eau) à la formulation concentrée de néogoutte (telle que fabriquée dans l'exemple 2 ci-dessus). L'addition du solvant de dilution peut être faite sous agitation (e.g. par turbine), ou l'agitation peut être effectuée après l'addition du solvant de dilution.

Ainsi, toutes les néogouttes décrites présentement peuvent être diluées dans des solutions aqueuses, par exemple au 1/5^{ème}, 1/10^{ème}, 1/20^{ème} ou encore 1/50^{ème} (voir procédure détaillée ci-dessous).

Par exemple, les néogouttes de l'exemple 2 peuvent être diluées au 1/10^{ème} dans une solution aqueuse. Une solution aqueuse est alors ajoutée sous agitation à la formulation obtenue selon l'exemple 2 jusqu'à ce que la solution initiale soit diluée au 1/10^{ème} (i.e. 9 volumes de solution aqueuse de dilution ajoutés à 1 volume de la formulation selon l'exemple 2).

### Exemple 4 : Tests comparatifs

Dans le cadre de la présente invention, un test clinique comparatif entre des néogouttes sans lauroyl-SDKP-OH et des néogouttes avec Lauroyl-SDKP-OH a été effectué sur des volontaires saints.

Dans le cas des formulations comprenant les néogouttes sans Lauroyl-SDKP-OH, la portion manquante de Lauroyl-SDKP-OH dans les néogouttes a été comblée par du « Myrj S40 ».

La quantité de Lauroyl SDKP-OH dosée dans les néogouttes dans le produit fini utilisé dans le cadre de ces expériences était inférieure à 1.10⁻⁵% en masse par rapport à la masse totale de formulation.

Néanmoins, même à ces taux de concentration, des effets physiologiques ont été rapportés.

### 1) Effet lissant

Un effet lissant significatif a été mesuré vs placebo dans 75% des cas dès 14 jours avec une rugosité moyenne diminuée de 11%.
*Variations jusqu'à -19%*

### 2) Effet anti-rides

Un effet anti-rides significatif a été mesuré vs placebo dans 81% des cas dès 14 jours et 77% à J28 avec une variation du relief de -14% et -16% respectivement.
*Variations jusqu'à -59%*

### 3) Effet restructurant / anti-âge

Un effet restructurant / anti-âge significatif a été mesuré dans 81% des cas à J14 et 69% à J28 avec une augmentation de l'isotropie de 12 et 14% respectivement.
*Variations jusqu'à +28%.* Ces résultats sont résumés sur la figure 1.

### 4) Effet redensifiant

Un effet redensifiant a été mesuré chez 68% des cas à J14 et 90% à J28, avec une augmentation moyenne de la densité de la peau de respectivement 12% et 37%. Ces résultats sont résumés sur la figure 2.

### 5) Effet sur l'épaisseur de derme

Une augmentation significative de l'épaisseur du derme a été mesurée chez 72%des patients.

### 6) Effet Hydratant

Un effet hydratant significatif à été mesuré vs placebo
*Variations jusqu'à +20%*

### 7) Effet tonifiant

Une amélioration significative de la tonicité de la peau a été mesurée chez 53% des sujets. Ces résultats sont résumés sur la figure 3.

Ces résultats particulièrement encourageants montrent l'intérêt du choix de la technologie selon le brevet FR2991196A appliqué à une formule modifiée de SDKP selon la présente invention.

## Revendications

1. Conjugué peptidique de formule (I) suivante :
R₁-X₁-X₂-X₃-X₄-A₁-R₂ (I)
dans laquelle :
- R₁, étant du côté N-terminal, est un atome d'hydrogène, ou une chaîne grasse choisie parmi un groupe alkyle substitué ou non substitué, linéaire ou ramifié en C₁₃-C₅₀, un groupe alkyle-aryle substitué ou non substitué, linéaire ou ramifié en C₂₁-C₅₀, un groupe alcènyle substitué ou non substitué, linéaire ou ramifié en C₁₃-C₅₀, un groupe R₃-CO-, R₃-OCO- ou R₃-COO- dans lequel R₃ est un groupe alkyle ou alcènyle substitué ou non substitué, linéaire ou ramifié en C₁₃-C₄₉, les substitutions incluant F, Cl, ou tout hétéroatome ou groupe d'hétéroatomes lipophile tel qu'une guanidine ou un guanidinium,
- X₁ est un condensat de (L)-sérine,
- X₂ est un condensat de (L) et/ou (D) acide aspartique,
- X₃ est un condensat de (L) et/ou (D) lysine, ,
- X₄ est un condensat de (L) et/ou (D) proline,
- X₄ étant optionnellement absent,
- les liaisons reliant X₁ à X₂, X₂ à X₃ le cas échéant, et X₃ à X₄ le cas échéant, pouvant être des liaisons peptidiques ou pseudopeptidiques,
- A₁ est une liaison covalente, un groupement NH ou un atome d'oxygène,
- R₂ est un atome d'hydrogène, ou une chaîne grasse choisie parmi un groupe alkyle substitué ou non substitué, linéaire ou ramifié en C₁₃-C₅₀, un groupe alkyle-aryle substitué ou non substitué, linéaire ou ramifié en C₂₁-C₅₀, un groupe alcènyle substitué ou non substitué, linéaire ou ramifié en C₁₃-C₅₀, un groupe R₄-CO-, ou R₄-OCO- dans lequel R₄ est un groupe alkyle ou alcènyle substitué ou non substitué, linéaire ou ramifié en C₁₃-C₅₀, les substitutions incluant F, Cl, ou tout hétéroatome ou groupe d'hétéroatomes lipophile tel qu'un groupement guanidine ou guanidinium,
- R₁ et R₂ ne pouvant être conjointement des atomes d'hydrogène,
ou l'un de ses sels pharmaceutiquement acceptables.

2. Nanoparticule lipidique comprenant :
- un cœur constitué d'une phase lipidique (L₁);
- au moins un agent tensioactif comprenant une partie hydrophile et une partie lipophile ;
- une membrane interne entourant ledit cœur constitué d'une phase lipidique (L₂), comprenant la partie lipophile dudit agent tensioactif;
- une membrane externe, entourant ladite membrane interne, constituée d'une phase aqueuse (A₁) comprenant la partie hydrophile dudit agent tensioactif ; et
- au moins un conjugué peptidique de formule (I) selon la revendication 1, **caractérisé en ce qu'**au moins une chaîne grasse en C₃-C₅₀ linéaire ou ramifiée, saturée ou insaturée, est greffée à l'extrémité N et/ou C terminale du brin peptidique, par exemple par l'intermédiaire d'un groupement C=O et/ou d'un atome d'oxygène et ledit conjugué peptidique est tel que sa partie lipophile est dans la phase lipidique L₂ et sa partie hydrophile dans la phase A₁.

3. Nanoparticule lipidique selon la revendication 2 **caractérisée en ce qu'**elle comprend en outre le conjugué peptidique de formule Ac-SDKP-OH, Ac-SDKP-NH₂ ou un mélange des deux.

4. Nanoparticule lipidique selon l'une quelconque des revendications 2 ou 3 **caractérisée en ce que** le conjugué peptidique est de formule (I) telle que définie selon la revendication 1, dans laquelle :
- R₁, étant du côté N-terminal, est un atome d'hydrogène, un groupe alkyle substitué ou non substitué, linéaire ou ramifié en C₃-C₅₀, un groupe alkyle-aryle substitué ou non substitué, linéaire ou ramifié en C₈-C₅₀, un groupe alcènyle substitué ou non substitué, linéaire ou ramifié en C₃-C₅₀, un groupe R₃-CO-, R₃-OCO- ou R₃-COO- dans lequel R₃ est un groupe alkyle ou alcènyle substitué ou non substitué, linéaire ou ramifié en C₃-C₅₀, les substitutions incluant F, Cl, ou tout hétéroatome ou groupe d'hétéroatomes lipophile tel qu'un groupement guanidine ou guanidinium, et
- R₂ est un atome d'hydrogène, un groupe alkyle substitué ou non substitué, linéaire ou ramifié en C₃-C₅₀, un groupe alkyle-aryle substitué ou non substitué, linéaire ou ramifié en C₈-C₅₀, un groupe alcènyle substitué ou non substitué, linéaire ou ramifié en C₃-C₅₀, un groupe R₄-CO-, ou R₄-OCO- dans lequel R₄ est un groupe alkyle ou alcènyle substitué ou non substitué, linéaire ou ramifié en C₃-C₅₀, les substitutions incluant F, Cl, ou tout hétéroatome ou groupe d'hétéroatomes lipophile tel qu'un groupement guanidine ou guanidinium.

5. Nanoparticule lipidique selon la revendication 4 **caractérisée en ce que** R₁ est un groupe alkyle substitué ou non substitué, linéaire ou ramifié en C₆-C₃₀, préférentiellement en C₁₀-C₂₀, ou un groupe R₃-CO- dans lequel R₃ est un groupe alkyle ou alcènyle substitué ou non substitué, linéaire ou ramifié en C₅-C₃₀, préférentiellement en C₉-C₂₀, les substitutions incluant F, Cl, ou tout hétéroatome ou groupe d'hétéroatomes lipophile tel qu'un groupement guanidine ou guanidinium.

6. Nanoparticule lipidique selon l'une quelconque des revendications 2 à 5 **caractérisée en ce que** ladite nanoparticule lipidique comprend au moins l'un des constituants suivants:
- au moins un acide gras en C₅-C₃₀, éventuellement hydrogéné et/ou sous forme d'ester de glycol, dans la phase (L₁),
- un ester d'acide gras en C₁-C₃₀ de polyoxyéthylène (10 - 100) comme agent tensioactif,
- au moins un acide gras en C₅-C₃₀, éventuellement hydrogéné et/ou sous forme d'ester de glycol, dans la phase (L₂), et/ou
- au moins un conservateur par exemple de type phénoxyéthanol dans la phase (A₁).

7. Nanoparticule lipidique selon l'une quelconque des revendications 2 à 6 **caractérisée en ce que** le conjugué peptidique comprend la séquence (L)S-(L)D-(L)K-(L)P.

8. Procédé de fabrication d'une nanoparticule lipidique selon l'une quelconque des revendications 2 à 7, **caractérisé en ce que** ledit procédé comprend les étapes suivantes :
a. préparation d'une phase lipidique et d'une phase aqueuse, l'une au moins des deux phases comprenant un agent tensioactif, l'une au moins des deux phases comprenant le brin peptidique de formule SDKP, ou un analogue biologique de celui-ci de nature peptidique, greffé d'une chaîne grasse telle que définie selon l'une quelconque des revendications 2 à 7 ;
b. émulsification de ladite phase lipidique et de ladite phase aqueuse conduisant à la formation de nanoparticules lipidiques, éventuellement sous pression ;
c. récupération des nanoparticules lipidiques formées.

9. Formulation cosmétique comprenant une ou plusieurs nanoparticules lipidiques selon l'une quelconque des revendications 2 à 7.

10. Formulation cosmétique selon la revendication 9 **caractérisée en ce qu'**elle comprend entre 1 et 25% en masse de nanoparticules selon l'une quelconque des revendications 2 à 7.

11. Nano-émulsion comprenant :
- au moins une phase lipidique dispersée (L₃), et
- au moins une phase aqueuse continue (A₂) dans laquelle la phase aqueuse comporte au moins un tensioactif, préférentiellement de type polyalkoxylé,
**caractérisée en ce qu'**à l'interface entre la phase aqueuse (A₂) et la phase lipidique (L₁) se trouve au moins un conjugué peptidique selon la revendication 1 ou 2 et/ou **en ce que** ladite nano-émulsion comprend au moins une nanoparticule lipidique selon l'une quelconque des revendications 2 à 7 dans laquelle éventuellement les lipides de (L₁) et (L₃), ou (L₂) et (L₃) sont identiques.

12. Utilisation cosmétique non-thérapeutique de préférence par application topique, d'un conjugué peptidique selon la revendication 1, d'une nanoparticule lipidique selon l'une quelconque des revendications 2 à 7, d'une nano-émulsion selon la revendication 11 ou d'une formulation cosmétique selon la revendication 9 ou 10 pour restructurer ou préserver l'aspect de la peau, en particulier en profondeur au niveau dermique.

13. Utilisation cosmétique non-thérapeutique selon la revendication 12 **caractérisée en ce qu'**il s'agit d'une utilisation antivieillissement.

## Patentansprüche

1. Peptidkonjugat der folgenden Formel (I)
R₁-X₁-X₂-X₃-X₄-A₁-R₂ (I)
wobei:
- R₁, das auf der N-terminalen Seite ist, ein Wasserstoffatom oder eine Fettkette ist, ausgewählt aus einer substituierten oder unsubstituierten, linearen oder verzweigten C₁₃-C₅₀ Alkylgruppe, einer substituierten oder unsubstituierten, linearen oder verzweigten C₂₁-C₅₀ Alkyl-Aryl-Gruppe, einer substituierten oder unsubstituierten, linearen oder verzweigten C₁₃-C₅₀ Alkenylgruppe, eine R₃-CO-, R₃-OCO- oder R₃-COO- Gruppe, wobei R₃ eine substituierte oder unsubstituierte, lineare oder verzweigte C₁₃-C₄₉ Alkyl- oder Alkenylgruppe ist, wobei die Substitutionen F, Cl oder jedes Heteroatom oder jede lipophile Gruppe von Heteroatomen wie ein Guanidin oder ein Guanidinium umfassen,
- X₁ ein Kondensat von (L)-Serin ist,
- X₂ ein Kondensat von (L)- und/oder (D)-Asparaginsäure ist,
- X₃ ein Kondensat von (L)- und/oder (D)-Lysin ist,
- X₄ ein Kondensat von (L-) und/oder (D)-Prolin ist,
- X₄ gegebenenfalls fehlt,
- wobei die Bindungen, die X₁ mit X₂, gegebenenfalls X₂ mit X₃ und gegebenenfalls X₃ mit X₄ verbinden, Peptid- oder Pseudopeptidbindungen sein können,
- A₁ eine kovalente Bindung, eine NH Gruppe oder ein Sauerstoffatom ist,
- R₂ ein Wasserstoffatom oder eine Fettkette ist, ausgewählt aus einer substituierten oder unsubstituierten, linearen oder verzweigten C₁₃-C₅₀ Alkylgruppe, einer substituierten oder unsubstituierten, linearen oder verzweigten C₂₁-C₅₀ Alkyl-Aryl-Gruppe, einer substituierten oder unsubstituierten, linearen oder verzweigten C₁₃-C₅₀ Alkenylgruppe, eine R₄-CO- oder R₄-OCO- Gruppe, wobei R₄ eine substituierte oder unsubstituierte, lineare oder verzweigte C₁₃-C₅₀ Alkyl- oder Alkenylgruppe ist, wobei die Substitutionen F, Cl oder jedes Heteroatom oder jede lipophile Gruppe von Heteroatomen wie eine Guanidin- oder Guanidiniumgruppe umfassen,
- R₁ und R₂ nicht gemeinsam Wasserstoffatome sein können
oder eines seiner pharmazeutisch verträglichen Salze.

2. Lipidnanopartikel, umfassend:
- einen Kern bestehend aus einer Lipidphase (L₁);
- mindestens ein Tensid, umfassend einen hydrophilen Teil und einen lipophilen Teil;
- eine den Kern umgebende innere Membran, bestehend aus einer Lipidphase (L₂), umfassend den lipophilen Teil des Tensids;
- eine äußere Membran, die die innere Membran umgibt, bestehen aus einer wässrigen Phase (A₁), umfassend den hydrophilen Teil des Tensids; und
- mindestens ein Peptidkonjugat der Formel (I) nach Anspruch 1, **dadurch gekennzeichnet, dass** mindestens eine lineare oder verzweigte, gesättigte oder ungesättigte C₃-C₅₀ Fettkette auf das N- und/oder C-terminale Ende des Peptidstrangs gepfropft ist, beispielsweise über das Zwischenglied einer C=O-Gruppe und/oder eines Sauerstoffatoms, und das Peptidkonjugat so ausgestaltet ist, dass sein lipophiler Teil in der Lipidphase L₂ ist und sein hydrophiler Teil in der Phase A₁ ist.

3. Lipidnanopartikel nach Anspruch 2, **dadurch gekennzeichnet, dass** er ferner das Peptidkonjugat der Formel Ac-SDKP-OH, Ac-SDKP-NH₂ oder eine Mischung der beiden umfasst.

4. Lipidnanopartikel nach einem der Ansprüche 2 oder 3, **dadurch gekennzeichnet, dass** das Peptidkonjugat die Formel (I) aufweist, wie nach Anspruch 1 definiert, wobei:
- R₁, das auf der N-terminalen Seite ist, ein Wasserstoffatom, eine substituierte oder unsubstituierte, lineare oder verzweigte C₃-C₅₀ Alkylgruppe, eine substituierte oder unsubstituierte, lineare oder verzweigte C₈-C₅₀ Alkyl-Aryl-Gruppe, eine substituierte oder unsubstituierte, lineare oder verzweigte C₃-C₅₀ Alkenylgruppe, eine R₃-CO-, R₃-OCO- oder R₃-COO- Gruppe ist, wobei R₃ eine substituierte oder unsubstituierte, lineare oder verzweigte C₃-C₅₀ Alkyl- oder Alkenylgruppe ist, wobei die Substitutionen F, Cl oder jedes Heteroatom oder jede lipophile Gruppe von Heteroatomen wie eine Guanidin- oder Guanidiniumgruppe umfassen, und
- R₂ ein Wasserstoffatom, eine substituierte oder unsubstituierte, lineare oder verzweigte C₃-C₅₀ Alkylgruppe, eine substituierte oder unsubstituierte, lineare oder verzweigte C₈-C₅₀ Alkyl-Aryl-Gruppe, eine substituierte oder unsubstituierte, lineare oder verzweigte C₃-C₅₀ Alkenylgruppe, eine R₄-CO- oder R₄-OCO- Gruppe ist, wobei R₄ eine substituierte oder unsubstituierte, lineare oder verzweigte C₃-C₅₀ Alkyl- oder Alkenylgruppe ist, wobei die Substitutionen F, Cl oder jedes Heteroatom oder jede lipophile Gruppe von Heteroatomen wie eine Guanidin- oder Guanidiniumgruppe umfassen.

5. Lipidnanopartikel nach Anspruch 4, **dadurch gekennzeichnet, dass** R₁ eine substituierte oder unsubstituierte, lineare oder verzweigte C₆-C₃₀ Alkylgruppe, bevorzugt C₁₀-C₂₀, oder eine R₃-CO- Gruppe ist, wobei R₃ eine substituierte oder unsubstituierte, lineare oder verzweigte C₅-C₃₀ Alkyl- oder Alkenylgruppe, bevorzugt C₉-C₂₀ ist, wobei die Substitutionen F, Cl oder jedes Heteroatom oder jede lipophile Gruppe von Heteroatomen wie eine Guanidin- oder Guanidiniumgruppe umfassen.

6. Lipidnanopartikel gemäß einem der Ansprüche 2 bis 5, **dadurch gekennzeichnet, dass** der Lipidnanopartikel mindestens einen der folgenden Bestandteile umfasst:
- mindestens eine C₅-C₃₀ Fettsäure, gegebenenfalls hydriert und/oder in Form eines Glykolesters, in der Phase (L₁),
- einen C₁-C₃₀ Fettsäureester von Polyoxyethylen (10 bis 100) als Tensid,
- mindestens eine C₅-C₃₀ Fettsäure, gegebenenfalls hydrierte und/oder in Form eines Glykolesters, in der Phase (L₂), und/oder
- mindestens ein Konservierungsmittel, beispielsweise vom Phenoxyethanol-Typ, in Phase (A₁).

7. Lipidnanopartikel nach einem der Ansprüche 2 bis 6, **dadurch gekennzeichnet, dass** das Peptidkonjugat die Sequenz (L)S-(L)D-(L)K-(L)P umfasst.

8. Verfahren zur Herstellung eines Lipidnanopartikels nach einem der Ansprüche 2 bis 7, **dadurch gekennzeichnet, dass** das Verfahren die folgenden Schritte umfasst:
a. Zubereitung einer Lipidphase und einer wässrigen Phase, wobei mindestens eine der beiden Phasen ein Tensid umfasst, wobei mindestens eine der beiden Phasen den Peptidstrang der Formel SDKP oder ein biologisches Analogon davon von peptidischer Natur, mit einer Fettkette aufgepfropft wie nach einem der Ansprüche 2 bis 7 definiert, umfasst;
b. Emulgierung der Lipidphase und der wässrigen Phase, die zur Bildung von Lipidnanopartikeln führt, gegebenenfalls unter Druck;
c. Gewinnung der gebildeten Lipidnanopartikel.

9. Kosmetische Formulierung, umfassend einen oder mehrere Lipidnanopartikel nach einem der Ansprüche 2 bis 7.

10. Kosmetische Formulierung nach Anspruch 9, **dadurch gekennzeichnet, dass** sie zwischen 1 und 25 Massen-% Nanopartikel nach einem der Ansprüche 2 bis 7 umfasst.

11. Nanoemulsion, umfassend:
- mindestens eine dispergierte Lipidphase (L₃) und
- mindestens eine kontinuierliche wässrige Phase (A₂), wobei die wässrige Phase mindestens ein Tensid umfasst, vorzugsweise vom polyalkoxylierten Typ,
**dadurch gekennzeichnet, dass** sich an der Grenzfläche zwischen der wässrigen Phase (A₂) und der Lipidphase (L₁) mindestens ein Peptidkonjugat nach Anspruch 1 oder 2 befindet, und/oder dadurch, dass die Nanoemulsion mindestens einen Lipidnanopartikel nach einem der Ansprüchen 2 bis 7 umfasst, wobei gegebenenfalls die Lipide von (L₁) und (L₃) oder (L₂) und (L₃) identisch sind.

12. Kosmetische, nicht therapeutische Verwendung, vorzugsweise durch topische Anwendung, eines Peptidkonjugats nach Anspruch 1, eines Lipidnanopartikels nach einem der Ansprüche 2 bis 7, einer Nanoemulsion nach Anspruch 11 oder einer kosmetischen Formulierung nach Anspruch 9 oder 10 zum Umstrukturieren oder Erhalten des Aussehens der Haut, insbesondere in der Tiefe auf dermaler Ebene.

13. Kosmetische, nicht therapeutische Verwendung nach Anspruch 12, **dadurch gekennzeichnet, dass** es sich um eine Anti-Aging-Verwendung handelt.

## Claims

1. A peptide conjugate of formula (I) below:
R₁-X₁-X₂-X₃-X₄-A₁-R₂ (I)
wherein:
- R₁, which is on the N-terminal side, is a hydrogen atom, or a fatty chain chosen from a substituted or unsubstituted, linear or branched C₁₃-C₅₀ alkyl group, a substituted or unsubstituted, linear or branched C₂₁-C₅₀ alkylaryl group, a substituted or unsubstituted, linear or branched C₁₃-C₅₀ alkenyl group, or an R₃-CO-, R₃-OCO- or R₃-COO- group wherein R₃ is a substituted or unsubstituted, linear or branched C₁₃-C₄₉ alkyl or alkenyl group, the substitutions including F, Cl, or any lipophilic heteroatom or heteroatom group such as a guanidine or a guanidinium;
- X₁ is an (L)-serine condensate,
- X₂ is an (L)- and/or (D)-aspartic acid condensate,
- X₃ is an (L)- and/or (D)-lysine condensate,
- X₄ is an (L)- and/or (D)-proline condensate,
- X₄ optionally being absent,
- the bonds linking X₁ to X₂, X₂ to X₃ where appropriate, and X₃ to X₄ where appropriate, possibly being peptide or pseudopeptide bonds,
- A₁ is a covalent bond, an NH group or an oxygen atom,
- R₂ is a hydrogen atom, or a fatty chain chosen from a substituted or unsubstituted, linear or branched C₁₃-C₅₀ alkyl group, a substituted or unsubstituted, linear or branched C₂₁-C₅₀ alkylaryl group, a substituted or unsubstituted, linear or branched C₁₃-C₅₀ alkenyl group, or an R₄-CO- or R₄-OCO- group wherein R₄ is a substituted or unsubstituted, linear or branched C₁₃-C₅₀ alkyl or alkenyl group, the substitutions including F, Cl, or any lipophilic heteroatom or heteroatom group such as a guanidine or a guanidinium group,
- R₁ and R₂ not being able to both be hydrogen atoms,
or a pharmaceutically acceptable salt thereof.

2. A lipid nanoparticle comprising:
- a core consisting of a lipid phase (L₁);
- at least one surfactant comprising a hydrophilic portion and a lipophilic portion;
- an internal membrane surrounding said core consisting of a lipid phase (L₂), comprising the lipophilic portion of said surfactant;
- an external membrane, surrounding said internal membrane, consisting of an aqueous phase (A₁) comprising the hydrophilic portion of said surfactant; and
- at least one peptide conjugate of formula (I) according to claim 1, **characterized in that** at least one linear or branched, saturated or unsaturated C₃-C₅₀ fatty chain is grafted at the N- and/or C-terminal end of the peptide strand, for example by means of a C=O group and/or an oxygen atom and said peptide conjugate is such that its lipophilic portion is in the lipid phase L₂ and its hydrophilic portion is in the phase A₁.

3. The lipid nanoparticle as claimed in claim 2, **characterized in that** it also comprises the peptide conjugate of formula Ac-SDKP-OH, Ac-SDKP-NH₂ or a mixture of the two.

4. The lipid nanoparticle as claimed in any one of claims 2 or 3, **characterized in that** the peptide conjugate is of formula (I) as defined in claim 1, wherein:
- R₁, which is on the N-terminal side, is a hydrogen atom, a substituted or unsubstituted, linear or branched C₃-C₅₀ alkyl group, a substituted or unsubstituted, linear or branched C₈-C₅₀ alkylaryl group, a substituted or unsubstituted, linear or branched C₃-C₅₀ alkenyl group, or an R₃-CO-, R₃-OCO- or R₃-COO- group wherein R₃ is a substituted or unsubstituted, linear or branched C₃-C₅₀ alkyl or alkenyl group, the substitutions including F, Cl, or any lipophilic heteroatom or heteroatom group such as a guanidine or guanidinium group, and
- R₂ is a hydrogen atom, a substituted or unsubstituted, linear or branched C₃-C₅₀ alkyl group, a substituted or unsubstituted, linear or branched C₈-C₅₀ alkylaryl group, a substituted or unsubstituted, linear or branched C₃-C₅₀ alkenyl group, or an R₄-CO- or R₄-OCO- group wherein R₄ is a substituted or unsubstituted, linear or branched C₃-C₅₀ alkyl or alkenyl group, the substitutions including F, Cl, or any lipophilic heteroatom or heteroatom group such as a guanidine or guanidinium group.

5. The lipid nanoparticle as claimed in claim 4, **characterized in that** R₁ is a substituted or unsubstituted, linear or branched C₆-C₃₀, preferentially C₁₀-C₂₀, alkyl group or an R₃-CO- group wherein R₃ is a substituted or unsubstituted, linear or branched C₅-C₃₀, preferentially C₉-C₂₀, alkyl or alkenyl group, the substitutions including F, Cl, or any lipophilic heteroatom or heteroatom group such as a guanidine or guanidinium group.

6. The lipid nanoparticle as claimed in any one of claims 2 to 5, **characterized in that** said lipid nanoparticle comprises at least one of the following constituents:
- at least one C₅-C₃₀ fatty acid, which is optionally hydrogenated and/or in glycol ester form, in the (L₁) phase,
- a C₁-C₃₀ fatty acid ester of polyoxyethylene (10 - 100) as surfactant,
- at least one C₅-C₃₀ fatty acid, which is optionally hydrogenated and/or in glycol ester form, in the (L₂) phase, and/or
- at least one preservative, for example of phenoxyethanol type, in the (A₁) phase.

7. The lipid nanoparticle as claimed in any one of claims 2 to 6, **characterized in that** the peptide conjugate comprises the sequence (L)S-(L)D-(L)K-(L)P.

8. A method for producing a lipid nanoparticle as claimed in any one of claims 2 to 7, **characterized in that** said method comprises the following steps:
a. preparing a lipid phase and an aqueous phase, at least one of the two phases comprising a surfactant, at least one of the two phases comprising the peptide strand of formula SDKP, or a biological peptide analog thereof, grafted with a fatty chain as defined in any one of claims 2 to 7;
b. emulsifying said lipid phase and said aqueous phase, resulting in the formation of lipid nanoparticles, optionally under pressure;
c. recovering the lipid nanoparticles formed.

9. A cosmetic formulation comprising one or more lipid nanoparticles as claimed in any one of claims 2 to 7.

10. The cosmetic formulation as claimed in claim 9, **characterized in that** it comprises between 1% and 25% by weight of nanoparticles as claimed in any one of claims 2 to 7.

11. A nanoemulsion comprising:
- at least one dispersed lipid phase (L₃), and
- at least one continuous aqueous phase (A₂) wherein the aqueous phase comprises at least one surfactant, preferentially of polyalkoxylated type,
**characterized in that**, at the interface between the aqueous phase (A₂) and the lipid phase (L₁), there is at least one peptide conjugate as claimed in claim 1 or 2 and/or **in that** said nanoemulsion comprises at least one lipid nanoparticle as claimed in any one of claims 2 to 7 wherein optionally the lipids of (L₁) and (L₃), or (L₂) and (L₃) are identical.

12. A non-therapeutic cosmetic use, preferably by topical application, of a peptide conjugate as claimed in claim 1, of a lipid nanoparticle as claimed in any one of claims 2 to 7, of a nanoemulsion as claimed in claim 11 or of a cosmetic formulation as claimed in claim 9 or 10, for restructuring or preserving the appearance of the skin, especially in depth at the dermal level.

13. The non-therapeutic cosmetic use as claimed in claim 12, **characterized in that** it is an anti-aging use.


### LISTAGE DE SEQUENCE

<110> RPM DERMATOLOGIE
<120> COMPOSITION COSMETIQUE CUTANEE COMPRENANT UN PEPTIDE SDKP OU UN ANALOGUE DE CELUI-CI
<130> FR n° 1651328
<140> FR n° 1651328
   <141> 2016-02-18
<160> 9
<170> PatentIn version 3.5
<210> 1
   <211> 4
   <212> PRT
   <213> Sequence artificielle
<220>
   <223> SYNTHETIQUE
<400> 1
   Ser Asp Lys Pro
   1
<210> 2
   <211> 4
   <212> PRT
   <213> Sequence artificielle
<220>
   <223> ACETYLATION N TERMINAL
<400> 2
   Ser Asp Lys Pro
   1
<210> 3
   <211> 4
   <212> PRT
   <213> Sequence artificielle
<220>
   <223> ACETYLATION N TERMINAL ET AMIDATION C-TERMINAL
<400> 3
   Ser Asp Lys Pro
   1
<210> 4
   <211> 5
   <212> PRT
   <213> Sequence artificielle
<220>
   <223> SYNTHETIQUE
<220>
   <221> misc_feature
   <222> (1)..(1)
   <223> Xaa can be any naturally occurring amino acid
<400> 4
   Xaa Ser Asp Lys Pro
   1 5
<210> 5
   <211> 6
   <212> PRT
   <213> Sequence artificielle
<220>
   <223> SYNTHETIQUE
<220>
   <221> X
   <222> (1)..(1)
   <223> PALMITOYLE
<400> 5
   Xaa Lys Thr Thr Lys Ser
   1 5
<210> 6
   <211> 4
   <212> PRT
   <213> Sequence artificielle
<220>
   <223> ACETYLATION N TERMINAL
<220>
   <221> SD
   <222> (1)..(2)
   <223> S EN POSITION 1 ET D EN POSITION 2 SONT LIES PAR UNE LIAISON PSEUDOPEPTIDE [CH2-NH]
<400> 6
   Ser Asp Lys Pro
   1
<210> 7
   <211> 4
   <212> PRT
   <213> Sequence artificielle
<220>
   <223> ACETYLATION N TERMINAL
<220>
   <221> DK
   <222> (2)..(3)
   <223> D EN POSITION 2 AND K EN POSITION 3 SONT LIES PAR UNE LIAISON PSEUDOPEPTIDE [CH2-NH]
<400> 7
   Ser Asp Lys Pro
   1
<210> 8
   <211> 4
   <212> PRT
   <213> Sequence artificielle
<220>
   <223> ACETYLATION N TERMINAL
<220>
   <221> KP
   <222> (3)..(4)
   <223> K EN POSITION 3 AND P EN POSITION 4 SONT LIES PAR UNE LIAISON PSEUDOPEPTIDE [CH2-NH]
<400> 8
   Ser Asp Lys Pro
   1
<210> 9
   <211> 7
   <212> PRT
   <213> Sequence artificielle
<220>
   <223> SYNTHETIQUE
<220>
   <221> X
   <222> (1)..(7)
   <223> X en position 1 est un atome d'H, alkyle C13-C50, aryl-alkyle C21-C50, R3-CO-, ou R3-OCO-, où R3 est un alkyle C13-C49 ou alcènyle; X en position 3 est D or N; X en position 4 est K, R or ornithine, X en position 6 est une liaison, NH or O, X en position 7 est un atome d'H, alkyle C13-C50, aryl-alkyle C21-C50, R4-CO-, ou R4-OCO-, où R4 est un alkyle C13-C50 ou alcènyle;
<400> 9
   Xaa Ser Xaa Xaa Pro Xaa Xaa
   1               5